# EUROPEAN PATENT SPECIFICATION

(11) **EP 3 664 649 B1**
(45) Date of publication and mention of the grant of the patent: **21.10.2026**
(21) Application number: 18845275.9
(22) Date of filing: 06.08.2018
(51) Int. Cl.: A41D 13/015, A41D 13/05, A01K 97/06, C09J 175/08, C08G 18/10, C08G 18/22, C08G 18/48, C08G 18/76, C08G 59/16, C08K 5/00, C09J 4/00, C09J 191/00, C09D 175/08

(54) **ADHESIVE VISCOELASTOMER AND ITS USE IN STABILIZED STORAGE CONTAINERS**
HAFTENDES VISKOELASTOMER UND SEINE VERWENDUNG IN STABILISIERTEN LAGERBEHÄLTERN
VISCOÉLASTOMÈRE ADHÉSIF ET SON UTILISATION DANS DES RÉCIPIENTS DE STOCKAGE STABILISÉS

(30) Priority: 07.08.2017 US 201715731815
(43) Date of publication of application: 17.06.2020
(73) Proprietor: Tak Logic LLC, Ettrick, WI 54627 (US)
(72) Inventor: KRIESEL, Matthew Wayne, Melrose, WI 54642 (US); GOODENOUGH, Troy Bradley, Mindoro, WI 54644 (US)
(74) Representative: Barker Brettell LLP
(86) International application number: PCT/US2018/045460
(87) International publication number: WO 2019/032479

(56) References cited:
- US-A- 3 507 071
- US-A- 5 564 001
- US-A- 5 677 413
- US-A1- 2004 134 669
- US-A1- 2008 005 929
- US-A1- 2010 170 139
- US-A1- 2013 288 060
- US-A1- 2018 042 209

## Description

### TECHNICAL FIELD

The present invention relates to a highly cohesive and adhesive thermoset viscoelastomeric reaction product, and to stabilized storage containers, more particularly to storage containers equipped with inserts of the reaction product which securely maintain and release stowed items at their original placement.

### BACKGROUND OF THE INVENTION

Inserts for commonly used compartmentalized storage containers such as for kitchen appliances, tool boxes, tackle boxes, desk drawers, and the like, often rely upon non-slip rubber inserts or adhesively backed hook-and-loop type fasteners (e.g., VELCRO) to retain stowed items in an orderly manner. The dual hook-and-loop type fastener system requires a dual adhesive attachment to the item to be retained and to the container itself. Other means for retaining objects to containers include molded cavities or other mechanical retention means (*e.g*., clamps, straps, coverings, clips, etc.) designed to mechanically restrain an item within the container. Unfortunately, none of these container retention systems have effectively solved an age-old problem of effectively retaining stowed items in their originally stowed position. Too frequently, such retention systems, such as upon use or aging, become broken and/or inoperable for practical use.

The age-old problem of orderly maintaining items stored in containers as originally stowed is well illustrated by the common use of fishing tackle boxes filled with fishing equipment (e.g., fishing lines, fishing lures, hooks, leaders, sinkers, etc.) which, upon use, often results in a snarled and entangled mess of fishing equipment. As a result, the fisherman often devotes precious fishing time to the tedious task of untangling and re-prepping the desired fishing equipment. The disarray of tackle box items occasionally even leads to personal injury, such as an inadvertent hooking injury (*e.g*., puncture wound, laceration, etc.).

It is practically impossible to maintain an orderly tackle box under its common environmental usage. Too frequently, the fishing tackle boxes are roughly handled, shaken, placed in an inverted transport position and/or roughly transported, which inherently causes the disarray of fishing equipment contained therein. In addition, boating to a fishing site will also frequently create conditions inherently leading to a disarray of fishing tackle box contents. This often arises due to the wind, boating waves, and the like typically encountered as the fishing boat speeds atop water to the fishing site. Numerous attempts to solve this age-old problem have remained unsuccessful. A common practice of placing non-slip rubber mats in each of the individual tackle box compartments has failed to correct the problem since the mats, as well as the tackle box compartments themselves, provide little, if any, protection against displacement of stowed tackle box equipment.

From a practical standpoint, available tacky releasable adhesives generally lack sufficient adhesive and cohesive properties to effectively restrain items and/or lack essential cohesive and adhesive stability for stowage use. Indeed, a heretofore unknown problem associated with releasable adhesives involves the lack of sufficient adhesive stability of the adhesive material when under pressurized adhesive conditions. A further problem associated with such adhesives involves its inability to retain substantially the same degree of adhesiveness upon an adhered stowed item over an extended stowage period. Indeed, many such adhesives exhibit a continuous reduction in adhesiveness over time, while others convert into a permanent adhesive (e.g., a non-releasable glue-like material) rendering it virtually impossible to subsequently remove a stowed item adhesively bonded thereto.

Accordingly, there exists a need for a unique adhesive composition which can serve as a stabilized adhesive stowing insert that retains a substantially constant degree of adhesiveness and cohesiveness when used as an insert for stowing items over extended stowing periods. There further exists a need for a container insert which can maintain a stowed item at its original stowed positon, even if such item is subjected to disrupting forces (e.g., bouncing, shaking, tumbling, etc.) and/or is stowed in an unorthodox position.

Unexpectedly, it has been discovered pursuant to the present invention that a unique thermoset cohesive and adhesive viscoelastomeric reaction product, when fabricated into a stowing insert, provides a powerful and a substantially constant degree of adhesiveness and cohesiveness so as to positionally retain and release a stowed item over prolonged stowage periods.

The present invention solves the perplexing age-old problem of effectively retaining articles in their original stowed position for subsequent use by a unique container insert derived from a unique thermoset reaction product which can be bonded to the container or can serve as an adhesive insert between the contacting surfaces of the container and a stowed item. Consequently, items placed within the container and onto the insert will remain in their original immobilized position under a substantially stabilized adhesive attraction by the insert notwithstanding subsequent physical or environmental mishandling. Thus, for example, when a fisherman arrives at a fishing site, everything within a tackle box equipped with the unique inventive insert will remain as originally placed for instant retrieval and use.

As demonstrated by the non-limiting examples described above, there accordingly exists a long standing need for a unique adhesive composition useful in the manufacture of a unique bonded or removable insert possessing powerful cohesiveness and adhesive or bonding attraction such as to a container wall (*e.g.,* top, ceiling, sidewall or bed walls, bottom, etc.), as well as to a wide variety of items placed thereupon for stowage therewith. There is also a need for an adhesive composition, and an insert resulting therefrom, to compositionally possess an inherent cohesiveness throughout its entire compositional make-up. There still further exists a need for a unique adhesive and cohesive thermoset composition and/or insert which, upon extended use, may be cleansed of interfacing adhesively clogging debris (e.g., dirt, dust, grime, etc.), such as by simply removing the insert from the container and washing with common household detergents (e.g., dish soap and water, etc.) to restore its surface adhesiveness. There yet further exists a commercial need for a self-cohesive composition and/or insert which possesses sufficient adhesive tenaciousness to rigidly retain adhered articles placed thereupon, but which will also allow the articles to be removed therefrom without damaging or breaking the articles. There still further exists a need for a cohesive and adhesive composition and/or insert which, upon release of a stowable item, will separate cleanly without leaving any residue of the composition and/or insert upon the separated stowed item. There yet further exists a need for a composition and/or insert possessing antimicrobial properties capable of providing an aseptic stowing environment.

Unexpectedly, the unique cohesive and adhesive attributes of the thermoset viscoelastomeric reaction product, and container inserts comprising such reaction product, provided by this invention uniquely solve one or more of the needs described above.

US2013288060 discloses polyether-based two-component polyurethane adhesive compositions comprising castor oil, which are used for rotor blades for wind power plants.

### SUMMARY OF THE INVENTION

The present invention provides a unique thermoset viscoelastomeric reaction product which, when used as an insert for containers for example, creates a container with an unexpectedly superior ability to stow items. In contrast to containers fitted with non-slip inserts and/or other mechanical restraining systems, the present invention provides a unique thermoset viscoelastomeric reaction product having self-cohesive and adhesive properties having particular usefulness as an insert for stowing containers. These inserts derived from the inventive viscoelastomeric reaction product uniquely possess an inherent internal cohesiveness, as well as an adhesive attraction to both the container walls and to most stowable items. Consequently, cohesive and adhesive inserts, as provided by this invention, possesses a unique interfacial adhesive attraction to the container itself, as well as to the objects placed thereupon for stowage. Unlike conventional non-slip rubber type inserts which fail to possess any appreciable adhesive attributes, the inventive cohesive and adhesive inserts herein will typically compositionally possess an adhesive attraction of more than 300 grams of force (g_{f}) per square centimeter (cm²) of contact area as ascertained by a constant test speed pulling force needed to separate a thermoset viscoelastomeric reaction product test patch from a 1.76 cm² circular nickel plated test probe at 20°C. These unique adhesive attributes are well suited to maintain a sufficient adhesive attraction to securely maintain stowed items adhered thereto against any further inadvertent movement until a sufficient counter-acting force is applied to break the adhesive bonding between the stowed item and the inventive viscoelastomeric insert. Since the inserts are based upon a viscoelastomeric reaction product possessing powerful inherent adhesiveness and cohesiveness properties, they may be, upon extended use, prone to lose their surface adhesiveness over time due to potential external surface contamination of debris accumulation, such as dust particles, dirt, grime, and the like. However, the inventive thermoset viscoelastomers of the present disclosure may be provided in the form of a removable insert for container usage, which permits removal (*i.e.,* cleaning) of such potential contamination via conventional washing techniques, such as by placing contaminated inserts into a conventional household dishwasher, or by washing the inserts by hand using conventional household dish washing detergents mixed with water. Alternatively, if desired, the inserts may be permanently bonded to the container by thermoset bonding thereto (in which case the inserts can be wiped clean of any potential contaminants, such as by using a soap and water solution).

The present invention provides a unique thermoset viscoelastomeric reaction product possessing extraordinary cohesiveness and adhesiveness properties useful in solving the age old problem of maintaining an orderly arrangement of retrievable stowed items within the confinement of a container. The embodiments of the invention are particularly applicable for use in a stabilized container combination comprised of a container equipped with an insert of the inventive thermoset viscoelastomeric reaction product, which possesses an unexpectedly superior adhesiveness to the container walls (including sidewalls, bed and top walls) while also tenaciously adhesively retaining and immobilizing an item emplaced upon the insert. Surprisingly, the inventive inserts obtained from the inventive thermoset viscoelastomeric reaction product also possess unique antimicrobial properties. Indeed, the inventive viscoelastomer and inventive inserts fabricated therefrom compositionally possess their unique adhesive, cohesive and antimicrobial properties throughout their entire viscoelastomeric thermoset structure.

The inventive viscoelastomeric reaction product and inventive inserts thereof may be conveniently provided as removable sheets (*e.g*., inlays or liners) and/or as permanent coatings tenaciously bonded to the container walls (*e.g*., floor or bed, side and top walls) of a stowing container. Since the thermoset viscoelastomeric compositions used herein inherently possesses a highly tacky, adhesive and cohesive structure, the resulting viscoelastomeric insert, when in a removable form, will also remain tenaciously adhesively bonded to the container as well as to stowed items placed thereupon. The adhesion characteristics of the insert, however, do not permanently immobilize an item placed in contact therewith, but will adhesively disengage (without leaving any cohesive residue) via the application of a pulling force sufficient to disengage the stowed item from the thermoset insert. These unique cohesive and adhesive thermoset viscoelastomer characteristics permit a disengagement of those items placed upon the insert, as well as a selective removal of the insert from the container. Furthermore, the cohesive and adhesive characteristics are extremely stable against cohesive and adhesive degradation. The adhesiveness of the insert towards a stowed item remains substantially unchanged over prolonged stowage conditions. When used as an insert, the container interface is typically of a greater total surface contact area than the stowed object placed thereupon, which results in a stronger adhesive attraction at the stowing container interface than to the articles placed thereupon. Accordingly, the total contacting surface of the insert which interfaces with the stowing container surface(s) will generally exceed the overall contacting surface interfacing with the stowed object. Consequently, a total lesser cohesive force will exist between the insert and the item contacting surface than between the insert and the container wall interface, thus allowing for the item to be removed from the insert without removing the insert from the stowage container. In some alternative embodiments, the interfacing section can optionally be substantially permanently bonded to the container.

Numerous other features and advantages of the present invention will appear from the following description. In the description, reference is made to exemplary embodiments of the invention. Such embodiments do not represent the full scope of the invention. Reference should therefore be made to the claims herein for interpreting the full scope of the invention. Titles may be used for convenience and should in no way limit the scope of the invention. In the interest of brevity and conciseness, any ranges of values set forth in this specification contemplate all values within the range and are to be construed as support for claims reciting any sub-ranges having endpoints which are real number values within the specified range in question. By way of a hypothetical illustrative example, a disclosure in this specification of a range of from 1 to 5 shall be considered to support claims to any of the following ranges: 1-5; 1-4; 1-3; 1-2; 2-5; 2-4; 2-3; 3-5; 3-4; and 4-5.

### BRIEF DESCRIPTION OF DRAWINGS

The foregoing and other features, aspects and advantages of the present invention will become better understood with regard to the following description, appended claims and accompanying drawings where:
Fig. 1 is a perspective top view of a stabilized container combination comprised of a sectioned container equipped with a thermoset viscoelastomeric insert of the present invention;
Fig. 2 is a perspective view of the stabilized container combination shown in Fig. 1 in which one compartment section comprises a stowed item;
Fig. 3 is a perspective top view of a stabilizing container combination of this invention comprising a stowed item;
Fig. 4 is a schematic cross-sectional view of Fig. 3 along line A-A showing a tray coated with a stabilizing insert coating of the present invention; and
Fig. 5 represents a schematic drawing of an adhesion testing apparatus which may be used to test the adhesiveness and cohesiveness of the thermoset viscoelastomeric reaction product and inserts thereof.

Repeated use of reference characters in the present specification and drawings is intended to represent the same or analogous features or elements of the present invention.

### DEFINITIONS

It should be noted that, when employed in the present disclosure, the terms "a" and "an" are intended to mean "at least one" of any stated features, elements, integers, steps, components, or groups and are not intended to be limited to only one of such features, elements, integers, steps, components, or groups thereof, except where specifically stated as such. In addition, use of the phrase "at least one" is not intended to render other uses of the terms "a" or "an" to be limited to only one of a feature, element, integer, step, component, or group.

It should also be noted that, when employed in the present disclosure, the terms "comprises," "comprising" and other derivatives from the root term "comprise" are intended to be open ended terms that specify the presence of any stated features, elements, integers, steps, components, or groups, and are not intended to preclude the presence or addition of one or more other features, elements, integers, steps, components, or groups thereof.

These and other terms may be defined with additional language in the remaining portions of the Specification.

### TEST METHODS

### Time Sensitivity Test

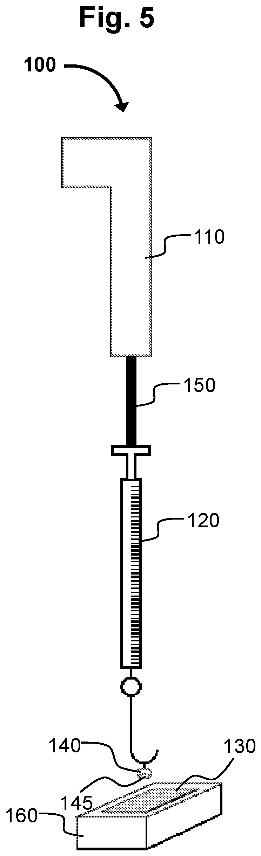

An uncured admixture (such as prepared in accordance with Example 1 below) is spread evenly at a uniform four (4) mm thickness upon a polyvinylchloride apron and allowed to cure into the desired thermoset viscoelastomeric reaction product. The resulting reaction product film is then cut into test panel patch sizes for testing using the testing apparatus depicted in Fig. 5 with the testing procedure being conducted in accordance with the Adhesive and Cohesive Test procedure set forth below, and further performing the optional *time dependent properties* procedures set forth therein..

### Adhesive and Cohesive Test

Referring to Fig. 5, the illustrated testing apparatus **100** is utilized for testing the adhesive and cohesive properties of various different inventive reaction products which had been formed into inventive thermoset viscoelastomeric inserts **30** of the present invention. The test apparatus **100** includes a motor driven actuator **110** (similar to that of a standard remote controlled electric garage door opener, such as a Model 3265, available from The Chamberlain Group, Inc., having a place of business located in Elmhurst, Illinois 60126, U.S.A.). The actuator **110** includes a reversible constant speed motor (2.7 cm/sec which is occluded from view) serving to drive a remotely controlled reciprocating test probe **150** connected to a measuring scale **120** to measure adhesiveness, which is the amount of applied force (in grams) needed to separate each thermoset viscoelastomeric insert test sample **130** from a polished nickel cylinder **140** having a 1.76 cm² contact surface area **145,** thus providing measurements in units of grams-force per square centimeter (g_{f}/cm²). A testing platform **160** of a laterally movable form is utilized to provide a solid flat level surface which allows for repositioning of the test sample **130** to provide an accurate repetition of the test results for each tested sample. The testing platform **160** may thus be repositioned to provide a repetition of an untested portion of the test sample insert **130** for further testing. Accordingly, adhesiveness is measured as the average of five (5) repetitions upon untested portions of a single insert sample **130.**

The testing procedure is also useful for determining the cohesiveness of the thermoset viscoelastomeric insert test sample **130.** This is accomplished by observing and noting the presence or absence of insert sample residue (*i.e*., reaction product residue) from each thermoset viscoelastomeric insert test sample **130** upon the test cylinder surface **145** after separating the cylinder **140** from the sample **130.** The cylinder surface **145** should be cleaned of any residue between each repetition, and the cohesiveness is measured as the average amount of residue over five (5) repetitions upon untested portions of a single insert sample **130.**

In addition, additional testing can include the application of pressure to the cylinder **140** when in contact with a thermoset viscoelastomeric insert test sample **130.** By measuring the adhesiveness and cohesiveness of each test sample **130** under differently applied sample application pressures, the adhesive and cohesive effects from applying such different adhesive pressures can likewise be determined. Similarly, adhesion separation differences or changes measured over timed sequenced intervals can also be determined so as to provide adhesive data upon an insert sample's **130** adhesion stability. The test procedure can also be utilized to provide adhesion data upon short interval adhesion increases following an initial adhesive attachment of the cylinder surface **145** to the test sample **130.** Differences in adhesiveness between pressure and non-pressure applied test probe over timed intervals can also be determined.

Continuing with Fig. 5, the following more detailed methodology was used to test adhesiveness and cohesiveness of various test samples **130:**
1. Scope
   1.1. This method measures the level of tackiness (adhesiveness) exhibited by adhesive materials and the stability (cohesiveness) of such materials, optionally taking into account time dependent and/or pressure dependent adhesive properties.
   1.2. This test is designed for use with materials that exhibit adhesive properties, but may also be used with materials not explicitly classified as adhesives, including but not limited to materials having adhesive-like properties.
   1.3. Units -The tested values of adhesiveness are based upon grams-force per square centimeter (g_{f}/cm²) of the force needed to separate the surface **145** of a polished nickel cylinder **140** having a contact surface area of 1.76 cm² from the test sample **130.**
2. Terminology
   2.1. As used herein, the term "adhesive-like" refers to having a sticky quality akin to an adhesive, but derives its sticky quality from molecular structure that forms a molecular attraction (rather than chemical bonded properties) which is releasable from adhered objects without leaving behind residue (*i.e.,* cohesiveness) . The test can be applied to an inventive thermoset viscoelastomeric insert test sample **130,** which additionally may be washed with a mild detergent and water to restore its stickiness when dirty.
   2.2. As used herein, the term "tackiness" refers to the quality of feeling sticky to the touch.
   2.3. As used herein, the term "time dependent adhesive" refers to a material in which adhesive strength increases according to duration of the contact time with a contacting surface. Such characteristic is distinctive from pressure sensitive adhesives since no substantial pressure is required to achieve the increased adhesive strength after initial contact.
3. Summary of Test Method Using the Testing Apparatus Depicted in Fig. 5
   3.1. An insert sample **130** of reaction product material to be tested is secured (generally via the insert sample's self-adhesive properties) to the testing platform **160.**
   3.2. The testing probe **150** of apparatus **100** is lowered to place the surface **145** of the cylinder **140** onto the sample **130.**
   3.3. The surface **145** of the cylinder **140** of the apparatus **100** remains in contact with the thermoset viscoelastomeric insert test sample **130** for a designated time period.
   3.4. The cylinder **140** of the apparatus **100** is raised from the sample **130** via the reciprocating probe **150** at a constant speed of 2.7 cm/sec to measure the force in grams required to separate the cylinder surface **145** from the sample **130,** as indicated by the measuring scale **120.**
   3.5. The measured separating force (adhesiveness) is then calculated and recorded in units of g_{f}/cm². In addition, the contacting surface **145** of the cylinder **140** is observed and the amount of residue attached thereto (if any) is recorded to determine cohesiveness. (Note: the surface should be cleaned if residue is present prior to further testing.)
   3.6. Steps 3.1-3.5 are then repeated on untested portions of the sample **130** so as to obtain a total of 5 tested samples, which are then averaged to yield a final result.
   3.7. Optionally, steps 3.1-3.6 can then be repeated over designated contact time intervals so as to determine the time dependent properties of the tested sample **130.**
   3.8. Optionally, steps 3.1-3.6 can then be repeated over designated applied pressures so as to determine the pressure dependent properties of the tested sample **130.**
4. Apparatus
   4.1. The testing apparatus **100** is illustrated in Fig. 5. Alternative materials and configurations to those stipulated may be used as long as they achieve comparable performance and meet the performance stipulations outlined in Section 4.2. Key elements of the apparatus include:
      4.1.1. A reciprocating mobile actuator **150** is responsible for lowering and raising the cylinder **140** onto the material sample **130** at a constant speed of 2.7 cm/sec.
      4.1.2. The scale **120** measures the amount of force in grams-force (per square centimeter) required to separate the contact surface **145** of the cylinder **140** from the test sample **130.**
      4.1.3. The contact surface **145** of the cylinder **140** is the sole contacting surface with the tested sample **130.** The contact surface **145** of the cylinder **140** is a circular polished nickel probe having a total contact surface area of 1.76 cm².
      4.1.4. The testing platform **160** provides a solid, level surface for accurate test results and secures the tacky test material sample **130** for testing. This platform **160** is laterally movable so as to allow for repositioning of the sample **130** for multiple testing.
   4.2. Regardless of the specific components used, the mobile actuator **150.**
      4.2.1. Raises and lowers the probe **TP** at a constant speed of 2.70 cm/second.
      4.2.2. The accuracy of testing apparatus and test measures force in grams with an accuracy resolution of five-percent (5%) or better.
      4.2.3. Except for optional pressure applied tests, a constant pressure of 20.0 g for the duration of the test was applied by the free-hanging, weighted cylinder **140.**
   4.3. The test procedure was conducted at ambient temperatures of 18°C to 24°C and most commonly at 21°C.
5. Calibration
   5.1. Prior to first use and at subsequent reasonable testing intervals afterwards, the speed rate of the actuator **150** is monitored to ensure consistency within the standard outlined in Section 4.2.1.
   5.2. Prior to first use, the accuracy of the scale **120** should be verified against a known weight and adjusted or zeroed accordingly.
6. Procedure
   6.1. Assemble the apparatus **100.**
   6.2. Secure a sample **130** of material to be tested to the testing platform **160,** ensuring that the platform **160** does not lift free during testing.
   6.3. Lower the testing cylinder **140** onto the test material **130,** ensuring even contact between the contact surface **145** and the test material **130,** and that the testing scale **150** is neither pulling nor applying pressure to the cylinder **140.**
   6.4. Allow the surface **145** of the cylinder **140** to remain in contact with the material sample **130** for the duration of the predetermined contact period.
   6.5. Continue the test by raising the surface **145** of the cylinder **140** from the test material **130.**
   6.6. Record the amount of gram-force as measured by the scale **150** needed to separate the surface **145** of the cylinder **140** from the test material **130.**
   6.7. Reset the scale **150.**
   6.8. Reposition the testing platform **160** so that a fresh (untested) area of the sample **130** is tested by the apparatus **100.**
   6.9. Clean the surface **145** of the cylinder **140** after each testing with a lint free cloth.
   6.10. Repeat steps 6.2 - 6.9 to obtain a total of 5 measurements.
   6.11. Optionally repeat steps 6.2 - 6.10 for each duration of testing to determine time dependent properties (e.g., at timed intervals of 15 seconds, 30 seconds, 5 minutes, 10 minutes and 15 minutes).
   6.12. Optionally repeat steps 6.2 - 6.10 for each duration of testing to determine pressure dependent properties (by applying incremental predetermined pressures to the cylinder **140).**
7. Calculation and Interpretation of Results
   7.1. Take each of the five (5) measurements, calculate the adhesiveness of the five (5) test sample **130** measurements by dividing the force (g_{f}) required for each sample **130** by the contact surface area **145** of the cylinder **140** (1.76 cm²), and then calculate the average of the five (5) adhesiveness measurements to established as a final value. The average tested value is given in the amount of gram-force per square centimeter (g_{f}/cm²) representing the required force to separate the surface **145** of the cylinder **140** from the material sample **130** which serves as a measurement of sample tackiness (adhesiveness).
   7.2. Take each of the five (5) observed residue quantities, and calculate the average of the five (5) quantities to establish a final cohesiveness value. A lower cohesiveness value can be more desirable than a higher value (e.g., wherein a lower value indicates less residue transferred to the contacting surface **145** of the cylinder **140** than a higher value).
   7.3. Repeat steps 7.1 and 7.2 for all iterations tested.

### DETAILED DESCRIPTION - Best Mode(s) - Industrial Applicability

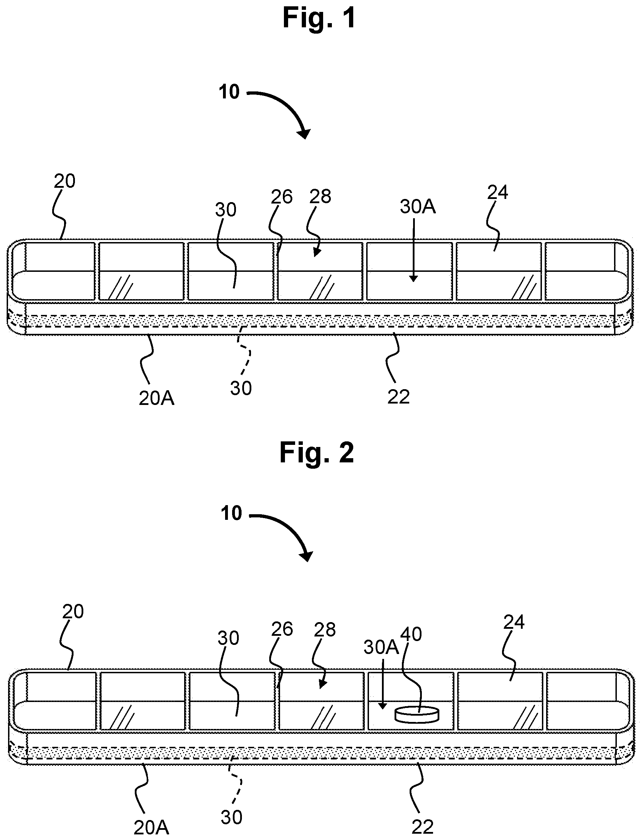

With reference to Figs. 1-5, there is provided pursuant to this invention a unique thermoset viscoelastomeric reaction product useful for use in a stowing container combination **10** equipped to uniquely stabilize emplaced articles or items **40** upon an insert **30** at a fixed stowable and stabilized emplacement position. This unique container combination **10** generally comprises a supportive base **20A** equipped with the unique cohesive and adhesive thermoset viscoelastomeric insert **30** derived from the reaction product which provides a first adhesive interfacial surface **30B** by adhesively adhering or by being bonded by thermosetting to the supportive base **20A** and an opposing adhesive surface **30A** positioned to adhesively engage and adhere onto a stowed article or item **40** emplaced thereupon. It has been unexpectedly discovered that the cohesive and adhesive thermoset viscoelastomer and the inserts **30** derived therefrom creates powerful and unique intermolecular cohesive and adhesive forces in what appears to be a localized intrinsic adhesive polarity charge or attraction which in turn provides an unexpectedly superior stowing adhesive efficacy when used as a container insert **30.** Compositionally, the cohesive and adhesive viscoelastomeric insert **30** effectively serves to stabilize and immobilize items or articles **40** against subsequent dislodgement until manual removal from the insert **30.** The stowing container combination **10** needs only a base support **20A** of either a solid or flexible construction in order to contain stowed items **40** since the exceptional cohesive and adhesive viscoelastomeric properties of the insert **30** serve to effectively restrain the items **40** adhesively placed upon the supportive base **20A** against any further movement.

Within the field of cohesive and adhesive materials, it has been found that there exists a very limited class of thermoset viscoelastomeric reaction products which meet the necessary prerequisital properties for use as an insert **30** as provided herein. Amongst the unique attributes most suitable for use as an insert **30** are:
1. a releasable adhesiveness which tenaciously clings onto an adhered stowed item **40** but will cleanly and cohesively release from an adhered item **40** without flaking or residue upon application of a counterforce sufficient to overcome the adhesive attraction between the stowed item **40** and the insert **30;**
2. a soft textured viscoelastomer which serves to embed an adhered item **40** within its adhesively engaging structure;
3. a viscoelastomer material of a substantially uniform cohesiveness throughout its entire compositional makeup providing a relatively high tensile strength which permits an adhesive embedding of an adhered item **40** with a clean cohesive separation therefrom when removed;
4. an adhesive material, which upon use, may be cleansed of adhering contaminates that adversely affect its adhesive efficacy;
5. a substantially stable insert **30** which retains a substantially stable degree of adhesiveness when stowing items **40** over prolonged storage periods; and
6. a thermoset viscoelastomeric reaction product and insert **30** characterized as having an adhesive adherence to stowed items **40** of more than 300 g_{f}/cm², as measured using the Adhesive and Cohesive Test procedure (described above).

The inventive insert **30** must compositionally possess a tenacious cohesiveness and adhesiveness in the form of a highly localized adhesive attraction to items **40** placed upon the insert **30.** Normal usage is typically effectuated by a slight pressing of the stowable item **40** against the insert **30** which in turn causes an indentation and embedding of the item **40** within the insert **30** along with a concomitant increase in overall interfacial contact area and an apparent localized intermolecular adhesiveness therebetween. Basically, the softer textured adhesive elastomers (*e.g*., soft rubber type cohesives) and particularly the thermoset viscoelastomers which possess the aforementioned prerequisitial cohesiveness and adhesiveness properties along with releasable attributes may be used for this purpose.

Due to the fluid flow characteristics of the thermoset reaction product under stress and its thermoset structure, the polymeric materials most suitably useful as inserts 30 in the combination 10 embrace those commonly referred to as a viscoelastomers. The thermoset viscoelastomeric reaction product and suitable inserts 30 for use herein broadly embrace a class of thermoset viscoelastomeric reaction products characterized as having a sufficient pliable character to embed stowed items 40 within its contacting surface area and a sufficient cohesiveness and adhesive attraction to tenaciously adhere to items 40 placed upon its surface 30A while also remaining substantially intact upon the removal of the stowed item 40 from the insert 30. Within the vast field of thermoset viscoelastomers, the newly discovered thermoset viscoelastomers have been found to possess surprisingly superior cohesiveness and adhesiveness rendering them most effective for use as inserts **30** herein. The unique stowing efficacy provided by the unique container combinations **10** fitted with the unique thermoset viscoelastomeric inserts **30** accordingly rests upon the unique cohesiveness and adhesiveness which the viscoelastomeric insert **30** compositionally and intrinsically provides to the combination **10.** It has been further observed that the viscoelastomeric insert **30** possesses an exceptional stability against any substantial cohesive or adhesive change over prolonged stowing time periods as well as uniquely providing antimicrobial properties.

Particularly effective are a novel class of the thermoset viscoelastomeric reaction products which unexpectedly possess a releasable but highly powerful cohesive and adhesive efficacy. Molecularly the thermoset viscoelastomeric reaction products apparently possess superior intermolecular cohesiveness and adhesiveness which renders the viscoelastomeric reaction product uniquely adaptable for use as a releasable insert **30** herein. The creation of such a unique thermoset viscoelastomeric reaction product and the inserts **30** derived therefrom compositionally involves providing a cured viscoelastomeric molecular structure which significantly contributes towards an increase in the adhesive tack as exerted by the insert **30** upon stowable items **40** coming in contact with its strong adhesive surface. This effect may be accomplished by creating a thermosetting reaction media which compositionally favors the development of a highly cohesive and adhesive thermoset viscoelastomeric reaction product for use as an insert **30.** The cured reaction media provides not only unexpectedly superior cohesive and adhesive attraction properties, but also other unique attributes which are significantly beneficial to the stowable container art. Apparently, the molecular makeup of the viscoelastomer and the unique adhesive and cohesive attributes of the inserts **30** will, upon engagement to an emplaced item **40,** provide an interfacing surface characterized as having apparent surface asperities and/or protuberance which effectively allows for an increase in the total area of contact between the item **40** and the insert **30.** This phenomena in combination with a powerful adhesiveness and an embedding of the item **40** within the insert **30** (*e.g.,* as due to viscoelastomeric properties) results in an unexpectedly powerful adhesive bonding and restraint of the item **40** to the insert **30.** In general the adhesive bonding of the item **40** to the insert **30** may actually be substantially greater than the gravitational forces exerted upon the item **40** itself as evidenced by an adhesive retention of an item **40** by the insert **30** even when the adhered item **40** is inverted to an upside-down, gravity-defying position.

The viscoelastomeric thermoset composition is derived from a unique thermosetting reaction media containing a cohesive mass of plasticizing agents structurally supported by a thermoset polymerization which provides an appropriate polarized level of molecular cross-linkage and bridging of straight chain linkages within the thermoset viscoelastomeric reaction product. The subsequently cured chemical viscoelastomeric thermoset derived from such a unique reaction media possesses a uniquely superior adhesive ability to cling onto contacting articles or items **40** with an intrinsic cohesive property when properly fabricated to selectively release adhered objects or items **40** therefrom by applying an outwardly pulling force sufficient to overcome the tenacious adhesive forces bonding the item **40** to the viscoelastomeric thermoset insert **30.** The adhesive separation breaks cleanly, leaving substantially no residue, since the thermoset viscoelastomer inherently possesses sufficient internal cohesiveness to tenaciously retain its structural integrity.

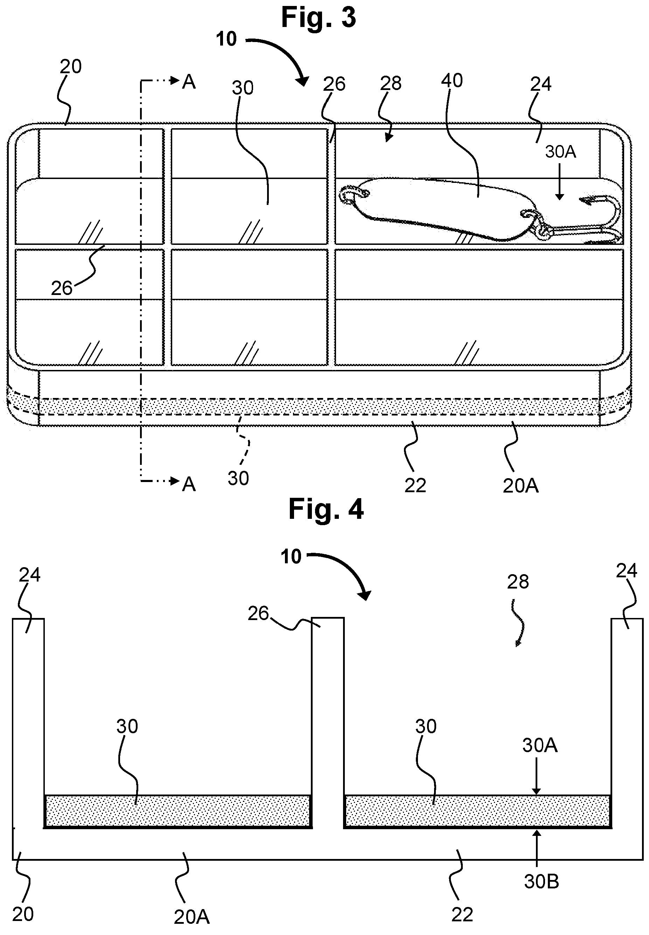

The unique embodiments of the thermoset viscoelastomeric insert **30** and the stabilized stowing container combinations **10** herein are illustrated in Fig. 3 as a container **20** in the form of a transparent compartmentalized fishing tackle box portion and in Figs. 1-2 as a container **20** in the form of a pill box, each equipped with compartmentalized sections **26** having a container bed **22** fitted with an antimicrobial, adhesive and cohesive thermoset viscoelastomeric insert **30** which may be a removable insert or a tightly bonded insert **30** securely affixed via thermosetting to the supportive base **20A** (which such supportive base **20A** can alone serve as a container **20** in other aspects). In the combination **10,** the larger surface contact area of the viscoelastomeric insert **30** will typically provide a greater adhesive attraction to a supportive base **20A** (depicted as a container bed **22)** than the lesser total surface contact area typically afforded by an item or object **40** stowed thereupon. In contradistinction to conventional prior art containers typically equipped with rubber liners which serve only as non-slip liners to prevent surface slippage of stowed articles **40,** the inserts **30** of this invention atypically tenaciously adhesively adhere onto stowed articles **40** emplaced thereupon and retain their original emplacement orientation over prolonged stowage. The inserts **30** provided herein have been found to possess such an unexpectedly high order of adhesiveness so as to restrain an adhered article from dislodgement even when the container **20** with the adhered article **40** is placed in an inverted position. Surprisingly, such a gravity-defying phenomenon applies to hefty stowed articles **40** such as a heavy solid stainless steel dinner knife **40** or household scissors which would normally be expected to be readily dislodged from such an insert when placed in an inverted position.

Since the inventive thermoset viscoelastomeric inserts **30** characteristically possess a flexible structure, they are readily adaptable for use in combination with a supportive base **20A** of a flexible structure as well as those of a rigid support construction. Due to the unique and superior cohesive and adhesive efficacy of the viscoelastomer, the thermoset viscoelastomeric inserts **30** may be provided in any form which provides sufficient support and interfacial surface contact so as to adhere (physically or chemically) to a host of supportive substrates **20A** including the sidewalls **24,26,** top walls (*i.e.,* cover or ceiling) (not shown) and bed walls **22** of a container **20,** as well as flexible substrates (such as used in sporting goods), and also flat surface supports (*e.g*., countertops, trays, benches, tables, etc.) while also providing a sufficient interfacial adherence to any item **40** so as to confine or immobilize any item **40** placed in contact therewith. The necessary interfacial adherence of the thermoset viscoelastomeric insert **30** to a container structure (*e.g*., **22,24,26,** etc.) and stowable item(s) **40** may be effectuated by providing an insert **30** of sufficient size to immobilize those items or articles **40** desired to be stowed by the stabilized container combination **10.**

The thermoset viscoelastomeric reaction product may be suitably manufactured under unique thermosetting reaction media conditions in which the necessary thermosetting precursors of polymerizable reactants are immersed in a lipophilic plasticizing media, which upon curing, provides the desired thermoset viscoelastomeric reaction product which is especially suited for use as the viscoelastomeric thermoset insert **30** herein. In procedures which involve a reaction media thermoset bonding of the insert **30** to the container **20,** the uncured thermosetting reactants may be applied by spray coating, injection molding, casting, and the like, and/or combinations thereof, at a desired thickness to the desired container substrate (*e.g.,* walls **22,24,26** or container compartments **28)** and then allowed to cure *in situ* to provide a coated thermoset viscoelastomeric insert **30** which then may become essentially permanently bonded to the container **20.** The amount of plasticizer and the level of cross-linking precursors in the reaction media can change the cured bonding characteristics. This technology may be applied to the most commonly available containers **20** (*e.g*., containers comprising metal, as well as containers comprising non-metal such as wood, cloth, thermosets and thermoplastics, and the like). By applying the uncured reactants to the desired container sections (*e.g*., beds **22,** walls **24,26,** top walls, etc.), the uncured thermoset viscoelastomeric insert coating **30** when cured becomes more firmly bonded to the container surface(s) and may be used to provide a permanently bonded coating. Conventional flaming techniques to prepare a thermoplastic surface for coating may be applied to improve the bonding.

The uncured thermosetting reactants may accordingly be directly deposited (*e.g*., sprayed, injected, molded, casted, etc.) onto any suitable flexible or solid supportive base **22** (*e.g.,* container beds **22,** walls **24,26,** top walls, etc.) and allowed to cure into the desired thermoset bonded viscoelastomeric insert or coating **30.** Thus, the uncured thermosetting reactants may be applied and cured as coatings, strips, systematic castings (*e.g*., circles, spirals, etc.) or in any other fabricating form sufficient to create a thermoset viscoelastomeric insert **30** of a sufficient size to hold or immobilize the intended items **40** to be placed thereupon. Injection molding, extrusion, spraying, vacuum molding, blading, spreading and other conventional thermosetting coating techniques may be used to apply the uncured reactants to the container walls (*i.e.,* container beds **22,** walls **24,26,** top walls, etc.) as well as in the manufacture of prefabricated insert sheets adaptable for use as a removable insert **30.** The curing rate and flow characteristics (*i.e.,* viscosity) of the uncured reactants can be effectively controlled by the type and amount of catalytic agents, reactants, thermal conditions, plasticizers, etc. used in conducting the thermosetting reaction. Excellent viscosity control during the initial uncured prefabrication stages may be achieved, for example, by increasing the level of lower molecular weight plasticizers and a decrease of the higher molecular weight plasticizer (e.g., epoxidized soybean oil (ESO)) concentration.

The thermoset viscoelastomeric inserts **30** herein are particularly applicable for use to most portable or handled containers and may be prefabricated for use as an insertable or removable insert **30.** Such removable inserts may be prepared by conventional calendering, sheeting, molding, etc. of the uncured thermosetting reactants applied onto a suitable substrate at the desired insert thickness which upon curing may be subsequently cut to fit the desired compartmentalized container sections (e.g., container beds **22,** walls **24,26,** top walls, etc.).

The fishing tackle and medicinal pill boxes **10** (typically of a transparent construction) as shown in Figs. 1-3 embody several unique embodiments of the invention. As illustrated in Figs. 1-3, the sheet-like construction of the interfacing thermoset viscoelastomeric inserts **30** are shown as resting upon the container bed **22** within the compartmentalized container units **28** of a fishing tackle box and a pill box container **20.** If desired, the adhesive character of the major interfacing surface for item **40** contact may be altered so as to more readily release an adhered item **40** while still maintaining adequate adherence to the bed **22** of the container **20.** Such surface changes may be implemented by providing a bottom surface of a greater surface area which adheres to the bed **22** as a substantially flat or smooth insert surface whereas the upper surface may be an irregular surface containing pronounced ridges and valleys designed to substantially reduce its surface contact area with the item **40** and its concomitant cohesiveness. This is generally not needed since the container insert **30** interface area will typically exceed the stowed item interfacing surface contact area. Since the insert **30** inherently possesses a powerful cohesiveness and tensile strength, a substantially greater separating force will be required to overcome the cohesive and adhesive bonding of the insert **30** to the container **20.**

Compositionally, the self-adhering inserts **30** as used herein differ from the conventional non-slip type of liners such as the commonly used thermoplastic rubber inserts, etc. which normally require an adhesive adjunct or other adhesive coating in order to satisfactorily adhere to a container surface. Unlike such conventional inserts, the inventive thermoset viscoelastomeric inserts **30** as used herein possess powerful cohesiveness throughout their entire compositional structure so as to retain structural integrity upon separation from an adhered item **40** while also tenaciously adhering until released to most of the common metals, thermosets, thermoplastic, and the like containers (both solid or flexible), as well as most stowable articles **40.** The cohesive molecular components of the inserts **30** are tenaciously and inherently bound throughout its entire compositional make-up rendering it virtually impossible to leave any compositional residue when an adhesively viscoelastomeric insert **30** is separated from a container **20** or upon a removal of a stowed item **40** from the insert **30.** Since the highly adhesive inserts **30** (as provided herein) possess unusually high adhesive bonding efficacy comparative to any commonly known and available adhesive materials, they may be effectively used as a top ceiling or sidewall insert **(30)** for both solid or flexible containers **20.** The inserts **30** tenaciously and uniquely adhere to the stowed items **40** as well as to a conventional container **20** which unique attributes permit a horizontal, vertical and inverted stowage of items **40.**

The insert **30** may be appropriately tailored so as to adhesively fit a particular compartmentalized section **28** of a container **20.** There exist special benefits for certain combinations **10** provided by a removable insert **30.** This will permit a removal of the insert **30** from the container **20** for cleaning, repositioning, etc. This constitutes a major advantage for many of the container combinations **10** and especially for those equipped with small stowing compartment sections **28.** As illustrated by Example 3, the removable insert **30** may be provided in a consumer friendly form which allows the consumer to purchase an insert **30** adapted for insertion and use in a used stowing container. Since the cohesive and adhesive inserts **30** securely adhere to a host of objects **40** which come into direct contact therewith, the thermoset viscoelastomeric cohesive insert **30** also tends to potentially accumulate dust, dirt, linen, and other contaminants coming in contact with its exposed surface. Excessive accumulations of such contaminants can dramatically reduce the adhesive efficacy of the exposed external surface of thermoset viscoelastomeric insert **30** to the extent it no longer possesses a sufficient capacity to effectively stabilize and immobilize objects **40** placed upon its interfacing surface. By providing a thermoset viscoelastomeric interfacing section or insert **30** of the container combination **10** in a removable or washable form (*e.g.,* a film, sheet, etc. form), any foreign matter clogging its cohesive efficacy may accordingly be readily removed and restored to an effective cohesive and adhesive use by washing or replacement. Uniquely the accumulation of such unwanted foreign matter upon the thermoset viscoelastomeric insert **30** surface area may be effectively removed by simply washing the insert **30** with common soap and water solutions, such as by simply washing the insert **30** in a conventional dish washer with common dishwashing soaps so as to restore its adhesive efficacy. The superior cohesiveness of the viscoelastomeric reaction product and the insert **30** allows for washing under conditions which would often cause other materials to disintegrate. The removable insert **30** form thus provides certain useful advantages over the permanent bonded or thermoset insert **30** form and vice versa. It is therefore evident that for certain end uses, the removable insert **30** form (*e.g.,* bulky containers with highly compartmentalized small open surface areas) may be best suited, whereas the thermosetting bonded form may best serve the combination **10** for access friendly flexible or washable containers **20.** Commonly, hand toted solid container combinations **10** such as illustrated by Figs. 1-3, as well as other flexible container combinations **10,** may benefit by the thermosetting of reaction media to the supporting base **20A.**

Although the providing of the viscoelastomeric insert **30** as an insertable and removable liner **30** may be desirable for ease of cleaning, the uncured viscoelastomeric reactants when cured *in situ* upon the walls **24,26** or bed **22** of a restraining container **20** or receptacle **20A** will create a tenacious and permanent and possibly a chemical bonding therebetween. For many container combinations **10,** the thermoset bonded form (*i.e.,* curing to the supportive base **20A)** may be more desirable than a removable form. Permanent bonding of the insert **30** to the walls **24,26** or bed **22** of a flexible container (*e.g*., backpacks, medical handbags, purses, duffel bags, sport shirts, hunting jackets, etc.) provides a combination **10** which may also be readily cleansed by washing the entire container combination **10** including the supportive container **20** with the insert **30** being permanently bonded or thermoset bonded thereto without any concern over insert **30** disintegration. For other applications wherein the supportive surface affords a sufficient flat open surface area to permit the insert surface to easily be washed and cleansed from air borne and other contaminants, the thermoset bonding of the insert **30** to the container **20** may be desirable. Since compositionally the insert **30** provides antimicrobial protection, the insert **30** becomes particularly useful should microbial contamination become a major concern to particular combinations **10** of its use. Since the antimicrobial component exists throughout the entire insert **30,** it will not be removed by washing.

The term "stowing container combination **10"** as used herein refers to the combination of the insert **30** and any supportive structure **20A** (*e.g*., container beds **22,** walls **24,26** and/or top walls, etc.) which in the stowing container combination **10** collectively provides a confining and stowing environment for a stored item **40** contained by the stowing container combination **10.** The stowing container combination **10** may include conventional side walls **24,26,** compartments **28** and covers (not shown), but which may become unneeded for many applications such as in stowing trays (*e.g.,* a support substrate **20A** only, such as a bottom wall **22** for example). The ability to eliminate conventional confining structures arises because of the uniquely powerful cohesive and adhesive properties of the insert **30.** The insert **30** may be in a prefabricated coating form (continuous or discontinuous) or by sheathing, molding, film, slab, sheet, casting or any other prefabrication forming method which takes advantage of its cohesive and releasable adhesive features in the combination **10.** As previously mentioned, the insert **30** may interface onto any supportive structure **20A** essentially as a permanent or thermoset bond thereto (*e.g., in situ* curing) or as a removable liner **30** relying upon the powerful cohesive and adhesive characteristics of the insert **30.** Since the adhesiveness of the thermoset viscoelastomeric insert **30** serves to confine an adherent item **40** emplaced thereupon, the stowing container combination **10** may solely rely upon an open, flat supportive platform structure **20A** so as to avoid the need for conventional compartmentalized **28** containers **20** or other conventional container restraints. Accordingly, flat and open supportive structures **20A** such as a tray (having no side or top walls) in combination with the insert **30** uniquely provides the containing or confining container **20** combination **10** for stowed items **40.**

For certain combinations **10** as illustrated by the schematic cross-sectional tray **20A** depiction of Fig. 4, the thermoset viscoelastomeric insert **30,** when used in combination with a flat surfaced supportive substrate such as a tray **20A** or even as a flexible substrate fitted with the insert **30,** the insert **30** itself provides the confining mechanism for the stowing container combination **10** without necessitating any other further confining means such as conventional confining sidewalls or covers. The thermoset viscoelastomeric insert **30** thus adhesively bonds or chemically bonds to any adhesive compatible supportive base structure **20A.** The insert **30** effectively serves as a means to confine and stow an item **40** placed upon any suitable supportive base surface **20A** without the need of any other confining structure to retain the item **40.** Unlike conventional trays which typically require a physical restraint in order to restrain stowed items from unwanted dislodgement, the inventive inserts **30** provided herein by themselves serve as a powerful confining or restraining element for the stowed article **40** held in confinement within the combination **10.** Thus, any insert compatible supportive base **20A** in combination with the unique thermoset viscoelastomeric insert **30** provides the confining container **20** for the confined stowage of items **40.** The ability to contain stowed items **40** in a vertical or even an inverted position upon a flat supportive surface **20A** or an open flexible or supportive surface without needing any confining walls or other containment factor creates a unique innovation for the container industry. Due to its cohesive and adhesive efficacy of the viscoelastomeric insert **30** the need for a raised edge, rim or cover for a flat surfaced container combination **10** (*e.g.,* such as trays) accordingly becomes obsolete.

Due to its adhesiveness, the inserts **30** are prone to adhere microbes thereto in addition to stowable items **40.** The usefulness of the insert **30** becomes particularly applicable within the hygienic field such as typically arises in the common usage of dental and medical trays to retain stowable items **40** at medical and dental clinics, hospitals, examining rooms, etc. The inherent antimicrobial or aseptic properties of the insert **30** render it highly effective for a vast array of medical and other hygienic uses. Surgical trays, examining room trays, dental and dental hygienist trays, and the like equipped with the insert **30** to stow hygienic devices **40** afford a unique ability to immobilize surgical, medical and dental instruments placed thereupon while also providing a surprising and unexpected effective aseptic and sterile environment. Due to the unique antimicrobial properties of the thermoset viscoelastomeric insert **30** herein, the capacity to adhesively engage and stow medical and dental instruments in a sterile and aseptic environment represents a significant advance over the current hygienic state of the art. This is particularly significant since conventional adhesive materials lacking antimicrobial attributes in an unprotected environment create an unacceptable increased health hazard which common and unvoidable hazard is unexpectedly and effectively alleviated by the insert **30.** The permanency of the thermoset bonding or its removable features and exceptional viscoelastomeric insert tack or adhesive efficacy without needing further confinement, plus its antimicrobial properties, affords a significant innovative advance over the current state of the hygienic art.

The manufacture of the insert **30** in an unbounded form may be prepared by initially depositing the uncured thermosetting viscoelastomeric reactants in a suitable reaction media and prefabricating form (*e.g*., compartmentalized sized sections, or sheeted, etc.), such as by casting, injection molding, calendering, and the like, or by simply depositing measured amounts of the uncured flowable reactants upon a moving chemically inert belt (equipped with or without heating) etc. and thereafter allowing the uncured reactants to cure under manufacturing conditions adapted to form the thermoset viscoelastomeric insert **30** of a desired thickness and form. Upon curing in a desired calendered, strip, sheet, casted, etc. form, the cured thermoset viscoelastomeric reaction product may suitably be cut or sized to an appropriate configuration to match its desired container combination **10.** Inserts **30** of the desired configuration and thickness may also be prepared by conventional molding techniques (*e.g*., injection molding). For certain applications the appropriate uncured, flowable reactant dosage as needed to provide the desired insert configuration and coating thickness may be directly applied to the container **20** which then upon subsequent curing creates the desired stabilized container combination **10.** In the uncured thermosetting form, the thermoset reaction media can be formulated (*e.g.*, epoxidized soybean oil to ester plasticizer levels) so as to provide the desired flow or viscosity characteristics so as to permit the casting, molding, etc. of an interfacing coated section or insert **30** of a desired configuration and thickness.

The present invention may be broadly adapted to a broad range of stowing container combinations **10.** Combinations **10** can be designed for stowage of items **40** weighing a gram or less to those weighing a pound (about one-half kilogram) or more, all of which may be effectively stabilized and confined at their original stowed placement position. Notwithstanding the exceptional adhesive properties of the unique inserts **30** herein, the degree of adhesive immobilization exerted upon any item **40** placed upon the insert **30** will have a direct correlation to the total contacting surface area of the insert **30.** A greater interfacial surface contact area with the stowed item **40** generally leads to a greater total adhesion to items **40** so as to allow a placement for larger and heavier items **40** thereupon. The heavier and bulker items **40** may require thicker inserts **30** than those used to stow lighter items **40.** The adhesive characteristics or strength may vary over a wide range rendering it possible to match a desired degree of adhesiveness to a desired end use. The cohesive and adhesive inserts **30** used herein will characteristically possess sufficient viscoelastomeric properties which allow the stowed item **40** to be embedded or cradled by the insert **30** as opposed to merely compressing without any appreciable item **40** embedding, as is the case for hard rubber inserts. In addition to providing a sufficient degree of cohesiveness and adhesiveness, the inserts **30** used herein must necessarily provide a relatively high degree of cohesive and adhesive stowing stability. This adhesive stability is evident in that the adhesive attraction reaches 90% of its maximum adhesive tack within one minute after a stowed item **40** initial contacts the insert **30.**

The utilization of the thermoset viscoelastomeric inserts **30** provides a unique cohesive and adhesive environment creating strong cohesive and adhesive forces which lead to unexpectedly superior stowing advantages of the thermoset viscoelastomeric insert **30** over any other known container inserts. Conventional rubberized container inserts merely compress with a substantial and concomitant volume loss due to its compression. In contrast, when an item **40** is placed upon the inventive viscoelastomeric insert **30** with a normal amount of applied placement pressure to adhesively engage onto the item **40,** there will arise a concomitant counteraction of a fluidized flow by the insert **30** which in effect embeds the stowed items **40** within an adhesive cavity, thus creating a substantially greater contacting interface and adhesive attraction area than would normally arise by the simple tangential surface contact of a rubberized insert. It has also been observed that the adhesive attractive forces of the insert **30** will increase slightly after initial item **40** contact with the insert **30** and then stabilize. This same phenomena even applies when an item **40** is quiescently placed upon the insert **30** which placement may have a lesser initial adhesion release strength but with time will ultimately become stabilized to a comparable adhesive value as a pressure-applied item **40.** The unique and atypical stowed item adhesive environment as provided by the viscoelastomeric inserts **30** herein coupled with their superior adhesive efficacy create a stowing combination **10** possessing unheralded adhesion and adhesive stabilization upon stowed items **40** which were heretofore deemed unfeasible. Such an unheralded adhesive insert **30** advance allows for an uncanny confinement of a stowed item **40** in a horizontal, vertical or inverted position within the container combination **10.**

The unique cohesiveness and adhesiveness of the insert **30** may be partly attributable to the manner in which these viscoelastomeric inserts **30** inherently embed, cradle and adhesively engage a stowed item **40** coupled with their apparent extremely powerful adhesive attractive influence upon the stowed article **40.** Conventional elastomers generally compress unilaterally about an impinging article and unilaterally rebound upon decompression so that the resting article tends to primarily rest tangentially upon the conventional elastomer. In complete contrast, an article **40** placed upon the inventive viscoelastomeric insert **30** herein causes a displacement of the viscoelastomer while partially submerging into the surrounding viscoelastomeric mass without any substantive volumetric loss to the insert **30** (as opposed to compression of a conventional rubber elastomeric mass, for example). This creates an embedded cradle for the stowed item **40** while also applying an equalized pressure and adhesive attraction upon the stowed item **40.** The cradling caused by the inventive viscoelastomeric insert **30** exerts a uniquely uniform and powerful fluidized embedded and adhesive attractive force upon the embedded article **40.** It may accordingly be logically concluded that the viscoelastomeric insert **30** inherently imparts a significantly greater adhesive attraction when items **40** are adhesively embedded within the viscoelastomeric insert **30** for stowage.

The initial embedding by forcible placing of the item **40** onto the viscoelastomeric insert **30** seems to create an indentation of a significantly greater surface contact area and a fluidized adhesive environment which does not normally occur in a compressive force against a conventional elastomeric mass. This initiating contact establishes a deep seated adhesive and stabilized viscoelastomeric attraction subject to a stabilized increase in adhesion which in combination with what appears to be a molecular polar alignment within the cured thermoset viscoelastomeric reaction product exerts an extremely powerful adhesive attractive force upon the stowed item **40.** By physical analogy, it much easier to entrap an item by an encompassing cupping and fluid-like entrapment under superior attractive adhesive forces than to adhesively entrap and engage an item by tangential contact. It has also been further observed that pressure applied upon a stowable item **40** to more deeply embed the item **40** within the inventive insert **30** can cause a concomitant increase in the initial adhesive bonding force of the item **40** to the insert **30** by a factor of 10 % as opposed to a quiescent placement. However, the quiescent placement of an item **40** upon the insert **30** will ultimately stabilize to a comparable adhesive separation value (adhesiveness value) as when pressure has been applied to the item **40.** Evidently, there exists internal forces within the thermoset viscoelastomeric reaction product which creates this delayed increase in adhesiveness.

The surface area of the insert **30** facing the stowed object must also necessarily possess a releasable surface tack or adhesiveness which allows for an adhesive release of an item **40** placed thereupon as opposed to a permanent or temporary adhesive bonding between the article **40** and the insert **30.** Commonly stowed items **40** weighing substantially more than conventional fishing tackle lures, such as carpentry or mechanical tools as commonly stowed in tool boxes, can also benefit from a container combination **10** appropriately fitted with the unique stabilized adhesive and cohesive insert **30** which may be specifically tailor-made to meet its intended use. The tailoring of the adhesive value involves the extent of cross-linking and polyether linkage within the thermoset polymeric structure coupled with the amount and type of plasticizer provided within the thermoset viscoelastomeric reaction product. Moreover, the insert **30** must also provide a stable adhesive bonding without exhibiting any appreciable adhesive change over prolonged stowage conditions. Surprisingly, the inserts **30** as provided by this invention retains a substantially unchanged degree of adhesive bonding after stabilization with respect to items **40** stowed by the insert **30** over prolonged storage periods (*e.g.,* four (4) weeks or more). There typically exists less than a 10% adhesive change after the initial stowing contact of the item **40** with the insert **30.**

In contrast to conventional non-slip and rubber container inserts, the present inserts **30** typically exhibit an adhesion release strength of more than 300 g_{f}/cm² (grams-force per square centimeter) as needed to overcome the adhesive attraction between the insert **30** and the emplaced item **40** as determined by the Adhesiveness and Cohesiveness Test procedure (discussed above) which utilizes the testing apparatus illustrated in Fig. 5. If desired, inserts **30** exhibiting about a three-fold (900 g_{f}/cm²) to about a six-fold (1,800 g_{f}/cm²) or more in an adhesive release strength may be obtained. Combinations **10** possessing the unique stowage characteristics for most uses will typically embrace inserts **30** having an adhesive bonding strength of more than 400 g_{f}/cm², usually more than 500 g_{f} /cm², but for most generally less than about 1,200 g_{f} /cm² and especially those inserts **30** having an adhesive release or separation strength ranging from about 500 g_{f} /cm² to about a 1,000 g_{f} /cm². Even though the thermoset viscoelastomeric reaction product may be tailored too much higher releasable adhesiveness (e.g., greater than 1,200 g_{f} /cm²), the excessive adhesiveness can render it particularly difficult to release the item **40** from the container combination **10.** On a most practical use range, about 600 g_{f} /cm² to about 1,000 g_{f} /cm² represents a highly effective insert **30** usage range. Higher adhesive values become more difficult to manually separate and will tend to approach the permanent adhesive range. However, certain applications, such as using the inventive thermoset viscoelastomeric reaction product to securely ground a gaming apparatus to a grounded support (*e.g*., game play equipment, supportive posts, equipment anchoring, etc.), may make it desirable to utilize a reaction product of about 1,500 to about 2,200 g_{f} /cm² or higher adhesion and separation value.

As mentioned, the stowing container combination **10** need not be a rigid structure. Flexible stowing container combinations **10** such as flexible medical equipment containers, first responder tote bags, flexible photographic equipment containers, tool bags, fishing tackle bags and a host of other container types of a flexible construction may benefit from the unique stowage attributes afforded by this invention. Since the thermoset viscoelastomeric insert **30** tenaciously adheres to items **40** placed thereupon, the container combination **10** does not necessarily require traditional sidewalls or covering lids. Unlike conventional stowing containers which necessarily need to store stowed items in an upright position, the inserts **30** uniquely allow for storage of items **40** in a horizontal, vertical or inverted position without any substantial loss of adhesive attraction to the inverted item **40** over a prolonged storage time. Such surprising stowing attributes are indicative of the unique cohesive and adhesive stability provided by a container combination **10** fitted with the cohesive and adhesive viscoelastomeric thermoset insert **30** and the equivalents thereof as provided by this invention. The viscoelastomeric properties of the insert **30** will also provide stowed item **40** protection against impact damage.

In another embodiment of the invention. as illustrated by Figs. -1-4, the thermoset viscoelastomeric insert **30** may be utilized to impart a desired aseptic effect to the stowing container combination **10.** As illustrated by Fig. 2, compartmentalized pill boxes **20** for stowing medicinal pills are effectively aseptically protected from microbes through the use of an antimicrobial thermoset viscoelastomeric insert **30** which constitutes an inherent property of the thermoset viscoelastomeric reaction product. The pill box insert **30** (*see e.g.,* Figs. 1-2) inherently provides powerful aseptic properties to the container combination **10** and stowed items **40.** Thus, when the interfacing viscoelastomeric insert **30** (*e.g.,* as a coating, sheet, film layered section, etc.) is applied to a container wall **24,26** or bed **22,** both the exposed outer surface and the inner container interfacing surface abutting onto the insert **30** will inherently benefit from its unique anti-microbial properties. The thermoset viscoelastomeric insert **30** and its surrounding environment accordingly do not foster microbial growth, but rather inhibit the development of unhealthy microbial or fungal infestations. Accordingly, the use of the thermoset polymeric insert **30** for surgical and medical uses, as well as other hygienic uses, coupled with the inherent self-cohesive and uniform antimicrobial properties throughout its entire mass opens a new vista for aseptic sterilization which uniquely distinguishes the inventive insert **30** from any other conventional insert. Accordingly, medical and other hygienist containers **20** for retaining hygienic items **40** under aseptic conditions, such as those essential to the hygienic use of dental, medical or hygienist cabinet drawers and compartments, bags, kits, boxes including pill boxes, as well as flat surfaced combinations **10** such as surgical, examining, medical and dental trays, etc. all of which will typically include a supportive base **20A** interfacing onto and supporting the antimicrobial thermoset viscoelastomeric inserts **30.** Such a stowing container combination **10** exhibits unexpectedly superior and unique antimicrobial advantages over the current technology by maintaining an orderly, aseptic, sterile and systematic environment for a stabilized placement of sterile medical instruments, devices and medicinal compositions etc. all of which fulfills a long sought medical and hygienist need. As mentioned herein, should dust, debris and other contaminants (including airborne particle contaminants sanitized by the insert **30)** clog the adhesive surface of the thermoset viscoelastomeric insert **30,** the insert **30** may be readily restored to its original or native condition by a soap and water solution wash without adversely affecting its deep seated antibacterial, antifungal, adhesive and cohesive properties which uniformly permeate throughout the entire compositional makeup of the insert **30,** and those items **40** coming in contact therewith. Similarly to medicinal and dental applications, unwanted infections arising from germ infested fishing hooks and lures may also benefit through the use of the insert **30.** Thus, the thermoset viscoelastomeric inserts **30** are particularly well suited for a host of hygienic uses. Medical, dental and other hygienic combinations **10** such as used by First Responders, ambulance personnel, dentists, dental hygienists, physicians, surgeons, nurses, etc. are accordingly beneficially and uniquely bestowed with a superior self-sanitizing container combination **10** for stowing self-contained items **40** in a stabilized, immobilized, sterile and orderly environment.

If desired, the uncured insert reactants may be formulated so as to provide a transparent insert **30** which permits one or more locating indicia, labels, decals, written messages (such as item use) or other desired instructions, etc. which are clearly visible through the transparent insert **30.** Coloring additives such as pigments, dyes, and the like. may also be formulated into the uncured reaction media to provide a desired coloring effect. The insert **30** may similarly be color coded to identify the stowed items **40** immobilized within the confinement of the combination **10.** Similarly fragrances may also be formulated into the uncured reactants to provide a scented insert **30.** If desired, the insert **30** may also be externally utilized to cohesively and adhesively retain stacked or otherwise arranged containers or other items **20** in an orderly fashion. If desired, identifying coded coloring indicia may be incorporated into the insert **30** to identify the respective contents of each container **20** or compartment **28** of the combination **10.**

The insert **30** has a broad range of adhesive affinity to most customary containers **20** as well as to most stowable items **40** which broadly embraces a wide range of diverse stowed items and supportive containers **20,20A.** Accordingly, the insert **30** may be used to physically adhere or chemically bond a host of supportive bases **20A** and items **40.** The supportive base **20A** may be illustratively constructed of a wide range of diverse materials such as cellulosic materials (*e.g*., wood, wood composites, vegetative materials, nonwoven substrates, etc.), thermosets, thermoplastics, plastic composites, metals (*e.g*., aluminum, steel, tin, metal alloys, etc.) textiles, glass and a host of other adhesive compatible rigid or flexible supportive materials. Accordingly, the stowing container combination **10** broadly applies to any supportive base **20A** adhesively compatible with the inventive insert **30.** Certain of the halogen containing polymers (*e.g.*, PVC) for certain formulations are adhesively incompatible to the adhesive properties of the thermoset viscoelastomeric inserts **30** herein. This may be due to the electronegativity of the PVC and the thermoset viscoelastomeric reaction product which causes a repelling of like charges. Such non-adhesive PVC materials may, however, be effectively used as continuous and non-reactive manufacturing belts for use in the curing and prefabricating of molded, sheeted, filmed, casted, etc. inserts **30.**

The inventive insert **30** in its thermoset viscoelastomeric form may be appropriately provided in a non-toxic, environmentally friendly (green) form, virtually free from residual gases or volatizing gases. The insert **30** may also impart impact and vibration absorbing attributes which further serves to protect stowed objects **40** from impact and vibrational damage. Accordingly, fragile stowed items **40** such as a photographic lens, electronic equipment, circuit boards, glass items and other delicate items **40,** etc. may be protectively housed and/or restrainingly immobilized against impacting damage and injury. The viscoelastomeric reaction product may also be effectively combined as a coating insert **30** with a foamed rubber to provide a protective stowing wrap combination for protecting fragile items **40.** The thermoset viscoelastomeric insert **30** as desirably used in the container combination **10** will typically be less than 4 mm in thickness and more typically less than 3 mm. In the coated and thermoset bonded form, a substantially lesser amount of the viscoelastomeric insert **30** may be typically utilized for certain applications to provide the desired cohesiveness and adhesiveness. Typically, an insert **30** in a tightly bonded form or unbounded form will be less than about 60 mil (1.5 mm) in thickness and most typically fall within a thickness range of about 20 mil to about 50 mil (0.5 mm - 1.3 mm). Thicker sizes may be used but are generally unnecessary. Notwithstanding, the insert **30** will generally be sized to accommodate the container combination **10** and the stowed item **40.**

Traditionally polyurethanes are generally formed by reacting a polyol with an isocyanate, usually a di- or polyisocyanate such as the aromatic isocyanates (*e.g*., typically a diphenylmethane diisocyanate-MDI or tolune diisocyanate-TDI) and aliphatic isocyanates such as hexamethylene diisocyanate (HDI) or isophorone diisocyanate (IPDI) in a prepolymer form which serves as the polyurethane isocyanate reactant in a reaction media which may contain an epoxidized vegetable oil as the major reaction media component. The reactant components include the isocyanate prepolymers reacted diols and triols polyoxyakylene polyol prepolymers such as polyethers having terminal diol and triol groups (e.g., polyoxyethylene and/or polyoxypropylene diols and triols) typically of a molecular weight of more than 1,000.

The triols within the reaction media cause cross-linking at the three available triol groups whereas the diols provide an uncrossed-linkage or straight chain linkage within the thermoset polymeric structure. In order to achieve a highly effective stable cohesive and adhesive insert **30,** a proper proportional amount of cross-linkage and straight chain linkages is necessary. The thermoset polymeric structure necessitates somewhat of a lessened cross-linked structure along with a more pronounced presence of intervening straight chain polar attracting linkages to provide a more flexible viscoelastomeric backbone chain of an appropriate polarity for the hosting of the polarized plasticizers therein. This creates an intertwining backbone chain providing an excellent attractive force and cohesive polar alignment of plasticizing agent throughout the entire viscoelastomeric mass. Exemplary useful triols and diols have repetitive oxygen groups of the higher molecular chains such as the polyethers and polyesters containing either the polymerizable terminal diol or triol groupings. Since it is desirable to utilize polymerizable reactants which provide a lower workable viscosity for prefabrication of the curing reaction media into the desired manufactured form, the more fluid diols and triols (as well as plasticizers) provide a more easily workable viscosity range. The resultant reaction product of balanced cross-linkage and straight chain polarized linkages with a balanced plasticizer content homogeneously distributed within the polymeric network has been accordingly found to unexpectedly exhibit superior adhesiveness and cohesiveness rendering it particularly useful as an inventive insert **30** herein. The uncured reaction media when cured creates a thermoset chain, which when properly loaded with an effective amount of a plasticizer, becomes homogeneously distributed within the thermosetting reaction media to provide the desired cohesive and adhesive characteristics which renders the thermoset viscoelastomeric reaction product unexpectedly effective for use as an insert **30** herein. The adhesive and cohesive stability of the resultant thermoset reaction product requires a delicate balance between molecular cross-linkage and polar aligning straight chain thermoset moieties bridging structure including the ratios thereof, as well as the level and type of plasticizer and its content within the thermosetting reaction media. Generally, a lowering of the plasticizer content leads to a firmer and less tacky reaction product, while an excessively high diol reactant level can lead to a thermoset reaction product which, upon contacting a stowed item, becomes permanently bonded to the stowed item **40.** By an atypical reduced plasticizer content and a judicious control of the diol and triol ratio, a viscoelastomeric thermoset reaction product ideal for use as an inventive insert **30** is possible. The appropriate plasticizer content in combination with an increased polyether diol concentration within the thermosetting reaction media increases cohesiveness and tensile strength as well as thermoset softness. The increase in the proportionate amount of polyether diol to polyether triols in combination with the plasticizer provides adhesive stability which effectively permits the insert **30** to functionally possess a releasability of an item **40** when stowed over prolonged stowing times. The loading of the reaction media with a lower molecular weight polar plasticizer permits a reduction in the total plasticizer content, which in combination with an increased polyether straight chain linkage, provides an insert **30** possessing an unexpected stowing and releasability efficacy when used in the storage container combination **10.** The lower molecular weight plasticizers also tend to contribute to a more fluid curing reaction media which may be more easily fabricated into a cured reaction product of the desired prefabricated form.

When working with vegetable based oils, the reaction media desirably includes a measured amount of epoxidized oil component content (*e.g*., fatty triglycerides such as ESO) amounting to less than 50-percent of the total reaction media weight (but may still constitute a predominant reactant reaction media ingredient) along with an increase in the polymerizate level of the straight chain viscoelastomeric components (*e.g*., the polyether diols) within the thermosetting media. Surprisingly, by reducing the triglyceride oil (*e.g*., epoxidized) content and increasing the proportion of the straight chain forming thermoset polymerizable reactants, the cured tackiness of the insert **30** will dramatically increase even though the reaction media may still contain a triglyceride component (*e.g*., ESO-epoxidized soybean oil) as a predominant component. Increasing the compositional cohesiveness and adhesiveness generally entails decreased epoxidized oil content as well as a decrease in the cross-linking triol reactant leading to a substantially reduced cross-linkage in the polymeric structure with a concomitant increase in the straight chain producing diol reactant level which increases the intervening straight chain bridging structure within the thermoset viscoelastomeric molecule. This polymerizate molecular change (with or without the use of other conventional plastic plasticizers besides the epoxidized triglyceride) apparently creates a localized polymeric polarity charge effectively expressed by the unique oxygen containing viscoelastomeric polyether backbone structure intertwined within the cured reaction product mass leading to an effective polarized plasticizer loading ultimately resulting in outstanding adhesiveness and cohesiveness coupled with its ideal release properties. Effective cohesive and adhesive efficacy for the insert **30** may be achieved with a triglyceride content ranging from about 15-percent to less than 50-percent by weight of the total reaction media weight. The substitution or replacement of the epoxidized triglycerides with polar ester plasticizers (especially those of a substantially lower molecular weight) have been found to maintain a desired level of cohesiveness and adhesiveness while still retaining excellent releasability and stability properties. Certain polar ester plasticizers of a more fluid consistency at room temperature and typically of a relatively low molecular weight will contribute to ideal working viscosities during the initial curing stages rendering the reaction product and insert **30** prefabrication much easier. Typically, these more fluid ester plasticizers will have a molecular weight of 500 or less.

Illustrative of an insert **30** having an exceptional high degree of cohesive and adhesive efficacy in the container combinations **10** as depicted Figs. 1-4 may be appropriately prepared by selectively using a thermosetting precursor reaction media which provides a properly configured carbamate thermoset linkage (*e.g*., urethane) for the thermoset viscoelastomer. In general, the applicable thermosetting polyurethane precursor mixes for preparing the insert 30 will typically include a balanced amount of polyol prepolymers (*e.g*., diols and triols) reacted with a ring-opening species of a hardener (*e.g*., amines, amides, mercaptans, anhydrides, polycyanates such as a diisocyanate, etc.). The ratio of polyol reactants, hardeners, catalyst, reaction temperatures, etc. are pre-selected with an appropriate balance given to the diol and triol reactants to molecularly create the appropriate cross-linkage and linear linkage needed in combination with selective plasticizers and amounts to provide the unique stability, cohesiveness and adhesiveness of the thermoset viscoelastomeric reaction product for the insert 30. The use of reactants, catalysts, reaction temperatures, etc. causing excessively cross-linked thermoset polymers without sufficient straight chain polyether bridging between the cross-linkages are generally unsuitable to create the desired adhesiveness and cohesiveness for the unique viscoelastomer and the insert **30.** The thermosetting reactants are thus chosen so as to avoid an undesirably high degree of cross-linkage and a corresponding high glass transition temperature. Reaction conditions and reactants which favor a more linear, cohesive and adhesive viscoelastomeric thermoset backbone structure receptive to polar plasticizer loading are particularly well suited for use in providing the insert **30** herein. Since the more exothermic, elevated curing temperatures are more conducive to creating a more rigid thermoset; the reactants and reaction media selection, slower reaction rate catalysts, low curing temperatures, controlled curing times and triol cross-linking to diol straight chain producing reactants in combination with an effective plasticizer are precisely controlled in order to impart the desired insert **30** characteristics. Carefully controlled reaction media conditions coupled with the proper reactants and plasticizers will accordingly provide more flexibility, a lower degree of cross-linkage and a lower glass transition temperature to yield by a highly cohesive and adhesive thermoset reaction product. Apparently, the particular thermoset molecular configuration created by a proper balance between the cross-linkage precursors and the straight chain polymerizates configures the thermoset viscoelastomeric polymerizate to a form highly susceptible to effective plasticization with the appropriate polar plasticizer orientation being created within the thermoset viscoelastomeric labyrinth polymerizate structure to yield the desired unexpectedly superior cohesive and adhesive properties. The desired thermoset insert 30 will be further characterized as being a viscoelastomeric thermoset polymer exhibiting low rebound velocity and hysteresis properties in addition to unexpectedly superior cohesiveness and adhesiveness. Such thermoset viscoelastomers also characteristically exhibit excellent energy and attenuating properties capable of withstanding repetitive and prolonged shock stress without structural damage or any substantive sag or rebound loss.

Procedurally the reaction product preparation may illustratively involve thermosetting a reaction media homogeneously loaded with plasticizers which may include an epoxidized vegetable oil reaction media component as a predominant media weight but not as a major media component (e.g., more than about 15% but less than 50% of the total media weight) as well as other desired plasticizers, a carefully measured molar ratio of cross-linking polyols to straight chain producing polyether diols to reduce overall cross-linkages to create a desired bridging thereof with straight chain linkages, an isocyanate prepolymer hardener such as the aliphatic, aromatic, heterocyclic, etc. polyisocyanates, cycloaliphatic, arylaliphatic, isocyanates and an appropriate catalyst (*e.g*., slow acting along with catalyst). The reaction media contains an appropriate plasticizer loading specifically adapted to provides a curable reaction media, which upon curing, produces a viscoelastomeric reaction product having a unique polymerizate structure effectively loaded with polar oriented plasticizers uniformly or homogeneously distributed throughout its entire mass intertwined and supported by the thermoset polymerizate structure. Illustrative catalysts include tertiary amines, tertiary phosphines, strong bases (*e.g*., alkali and alkaline earth metal hydroxides, alkoxides and phenoxides), and the acidic metal salts of strong acids, metal chelates, metal alcoholates and phenolates, organic acid salts, organo metallic derivatives etc. Under the most desirable thermosetting and fabricating conditions, the polymerizate precursors and the plasticizers are provided in the reaction media as room temperature liquids without necessitating any solvents, other chemical dispersion aids or elevated temperatures to homogeneously disperse the reaction media components.

The diols and polyols of a relatively high molecular weight will effectively serve as thermoset viscoelastomeric cross-linking and straight chain building components for the intertwining thermoset viscoelastomeric polymeric structure which when properly balanced provide a plasticizer friendly viscoelastomeric polymeric structure of a well-balanced straight and cross-linked linkages of an appropriate polymerizate configuration and molecular polarity for plasticizer loading. On a reactant weight percentage basis, the diols are generally recognized as being less effective than the triols in producing shock absorbing polymeric structure, but are extremely effective herein in creating the desired viscoelastomeric polymeric structure necessary for plasticizer loading and imparting the desired unique adhesiveness, cohesiveness and release characteristics to the insert **30.** A judicious diol to triol balance enables a balanced proportion of polarized plasticizing components to effectuate the superior adhesiveness and cohesiveness within the carefully structured thermoset reaction product structure. To achieve the necessary plasticizing adhesive and cohesive efficacy, the straight chain diol precursors are typically of a relatively high molecular weight as may be provided by a polyether sequence which at increased reaction media concentration creates a more linear thermoset polymeric polyoxy structure while also serving to lessen the cross-linkage density. In general, the desired plasticity and flexibility along with the desired polarity, adhesiveness and cohesiveness may be accordingly effectively effectuated via interpolymerizing the proper amounts of the thermosetting higher molecular weight diols and triols of polyols (e.g., molecular weight of 2,000-10,000) along with other thermosetting cyanate reactants and plasticizers at the appropriate reaction media amounts. The useful polyether diols herein are characteristically comprised of a straight polyether molecular chain having two terminal hydroxyl groups. In contrast, the polyether triols characteristically have three terminal cross-linking reactive hydroxyl groups leading to more polyfunctional cross-linking sites in the thermosetting reaction media.

When using the ether polyols, the basic molecular structure of the thermoset reaction product typically requires a controlled cross-linked structure by balancing the dihydoxy to trihydroxy polyalkylene oxide ratio to create a thermoset viscoelastomer having a sufficient quantum of straight chain polyether linkages. The polar oxygen rich diol reactant serves to separate and becomes sandwiched between the cross-linking triol polymerizate linkages. The effective reduction in cross-linkage does not change its thermoset viscoelastomeric classification since its viscoelastomeric properties are retained. Apparently the controlled thermoset structure permeating the reaction mass creates a controlled polar density of cross-linked polyethers interspersed amongst straight polyether chain bridges to provide a unique labyrinth structure which apparently provides an appropriate polarity and allows for an effective loading of the necessary adhesive and cohesive contributing factors which include the use of plasticizer such as those commonly known by the trade as plastic plasticizers. By establishing a viscoelastomeric molecular structure loaded with properly oriented and entrained plasticizers, surprisingly superior adhesive and cohesive properties with stable release attributes are thereby achieved. Countless enclaves of negatively charged straight and cross-linked diols and triols appear to form a thermoset labyrinth loaded with polarized plasticizer uniformly distributed throughout the curing reaction media and the cured reaction product. This unique polarized polymerizate infrastructure and massive polarized plasticizer stacking within the polymerizate labyrinth apparently creates a synergistic adhesive and cohesive reaction product effect. The reaction product and the viscoelastomeric inserts **30** possess a surprisingly high tensile strength and softness indicative that its cohesive thermoset viscoelastomeric structure coupled with its plasticizer content significantly contributes towards a superior compositional cohesiveness and adhesiveness. Along with its high tensile strength, the reaction product and the viscoelastomeric insert **30** possess a high degree of elasticity.

The epoxidized vegetable oils may effectively serve as a plasticizer in combination with the bridging straight chain polyether diol polymerizate to create the desired cured flexibility, plasticization, releasability, cohesion and adhesion efficacy for the cured viscoelastomeric thermoset insert **30.** Particularly effective cohesive and adhesive properties arise when the epoxidized vegetable oil concentration range is less than 50-percent by weight with an amount less than 45-percent of the total reaction media weight providing further enhanced adhesive, cohesive and stability efficacy. The lower molecular weight plasticizers (*e.g*., particularly the less than 500 MW ester plasticizers) may be effectively used to replace the epoxidized vegetable oil plasticizer. Substitution of the epoxidized triglyceride with the ester plasticizers of a lower molecular weight will significantly increase fluidity and workability of the thermosetting reactants while still retaining the other desirable thermoset or insert attributes. Plasticizer (particularly the lower weight esters) coupled with a balanced triol and diol ratio can be effectively utilized to provide a reaction product and insert which will tenaciously cure and bond to a supportive base **20A.** The weight ratio of epoxidized triglyceride plasticizer to ester plasticizer (if present) may typically range from about 1:3 to about 3:1 and most typically between about 1:1 to about 3:1. The epoxidized vegetable oil (*e.g*., epoxidized soy bean oil, which is commonly referred by the trade as ESO) may typically comprise a predominant weight portion of the total reactant media with amounts ranging from about 20-percent to less than 30-percent by weight of the total reaction media weight being highly effective for certain applications. The molecular size and configuration, polarity, functional molecular groups etc. of the thermosetting polymeric reactants along with the combination of lower molecular weight ester plasticizer and the epoxidized vegetable oil plasticizer in measured amounts can be effectively used to contribute towards the creation of a desired insert **30** possessing the desired unique cohesive and adhesive properties. Although the epoxidized vegetable oil plasticizer may include a variety of epoxidized vegetable oils (*e.g.,* castor, corn, cottonseed, perilla, safflower, linseed, soybean, tall, etc.), epoxidized soybean oils have been found to be particularly effective as the epoxidized vegetable oil plasticizer component for preparing the reaction product and the thermoset viscoelastomeric inserts **30.**

Imparting the desired flexibility, adhesive and cohesive characteristics to an insert **30** compatible for interfacing onto the container confining walls (*e.g*., walls **22,24,26,** top walls, etc.) including the container bed **22** and stowed items **40** confined thereby may be illustratively prepared via utilizing a reaction media containing about 15-percent to about 30-percent by weight of a two functional polyether polyol (*e.g*., ELASTOCAST C-4057, available from BASF Corporation), about 15-percent to about 35-percent by weight of a three functional polyether polyol (*e.g*., ELASTOCAST C-4018, available from BASF Corporation), about 4-percent to about 10-percent by weight of methylene diphenyl diisocyanate based glycol prepolymer (*e.g*., ELASTOCAST TQZ- P23, available from BASF Corporation or sold by Dow Chemical as ISONATE 2181^{®} and RUBINATE 1790 ^{®} ), an epoxidized soybean oil in an amount ranging from about 25 -percent to less than 50-percent by weight of the total reaction media weight, along with a catalytic amount of a suitable catalyst (*e.g.,* and a Bismuth (3+) neodecanoate COSCAT 83, available from Vertellus Specialties) typically at a catalytic concentration ranging from about 0.1-percent to about 0.6-percent by weight of the total reactant weight. The polyether diols and triols are available at various chain lengths typically ranging from about 1,000 to about 20,000 molecular weight. The polyether diols and triols most suitably adaptable herein will have a molecular weight of less than 15,000 and most typically less than 10,000. Since there exists definitive advantages in maintaining fluidity during the initial mixing and prefabricating steps, the more fluid polyethers are more useful for most applications. Exemplary of the diol and triol reactants applicable herein include the polyoxyethlene and polyoxypropylene diols and triols thereof having a molecular weight generally ranging from 1,000 and 8,000. Particularly effective for use herein are the polyether diols within about 2,000 to about 6,000 molecular weight range and triols within about 2,000 to about 8,000 molecular weight range and most particularly from about 3,000 to about 5,000 MW. As mentioned, care needs to be exercised as to the extent of cross-linkage and intervening polyoxyalkylene linkage separating the cross-linking site. This is generally accomplished by retaining a diol to triol weight ratio content from about 7:13 to 13:7 and most typically from about 3:2 to about 2:3 to provide the desired straight chain polyether linkage and cross-linkage density.

As a reaction media component, the epoxidized vegetable oils are compositionally effective in providing adhesiveness, cohesiveness and plasticization for the insert **30.** The epoxidized triglycerides of vegetable oils also uniquely contribute towards the desired prerequisite viscoelastic properties while further imparting the desired cohesive attributes adaptable for use in combination with the confining container **20** and the items **40** desirably immobilized by the interfacing section **30.** As may be observed from the aforementioned formulations, the epoxidized vegetable oil may suitably constitute the predominant ingredient but at a level of less than 50-percent of the total weight of the uncured reaction media. Initially the uncured reaction media may most appropriately be formulated so as to possess sufficient flow characteristics to allow the uncured reactants to be pre-formed into a desirable cured shape for use as the insert **30** herein. The total plasticizer content will most typically fall within about 25-percent to about 45-percent by weight of the total reaction media weight. Superior adhesiveness and cohesiveness will most generally be accomplished by maintaining the total plasticizer content range from about 30-percent to about 40-percent of the reaction media weight.

In general, the cured thermoset viscoelastomeric composition prepared from uncured reactants of a high epoxidized soybean oil content will adhere to adhesively compatible polymeric materials such as the polyurethanes and PET (*e.g*., polyethylene terephthalate), the polyolefins (*e.g*., polyethylene, polypropylene), polyacrylates, etc. while other halogenated materials such as the halogenated polymers as exemplified by polyvinylchloride (PVC) will not be adhesively compatible (except for special formulations) but nonetheless provide excellent release properties which render such polymers particularly effective for use as a mold to cure the reactants. However, as illustrated herein and in the Examples, reaction medias designed to tenaciously adhere to normally non-adherent plastics upon curing may be prepared by a properly balanced reaction media.

Another observed unique insert feature resides in the manner in which the thermoset reaction product and the insert **30** will adhesively interact with items **40** adhesively attached thereto. This adhesive interaction is generally characterized by a slight initial increase in adhesiveness within about 5 seconds to about 10 seconds after its initial adhesive attachment to an item **40** which is then followed within 60 seconds by a stabilization to 90% of its maximum or ultimate adhesive attraction. This slight change in adhesiveness may be indicative of an intermolecular realignment, coordinate covalent bonding or polarization of the plasticizing components or other molecular interaction within the viscoelastomeric reaction product structure to provide for a delayed increase in adhesive attraction.

The preparation of the superior inventive cohesive and adhesive viscoelastomeric thermoset reaction product and its inventive inserts **30** necessitates controlling the cross-linkage at a level sufficient to retain its thermoset viscoelastomeric polymeric structure. Excessive molecular chain cross-linkage caused by the triol and higher polyhydroxyl components along with the isocyanate thermosetting reactants results in a viscoelastomeric structure that cannot be effectively loaded with an effective polarized orientation of the plasticizing component which is functionally essential to impart the superior cohesiveness and adhesiveness to the thermoset viscoelastomeric reaction product. If the viscoelastomeric cross-linkage is reduced too much, the polymeric structure is converted into a non-functional viscoelastomer or non-homogeneous thermoplastic mass which is molecularly and structurally incapable of retaining any cohesive and adhesive imparting components to a sufficient degree to provide a thermoset viscoelastomer possessing a releasable adhesive value of more than 300 gram-force per square centimeter (g_{f} /cm²). A properly balanced negatively charged enclave formed by monitored amounts of triol and diol polymerization precursors in the presence of a heavy load of polar orientable plasticizers effectively oriented within the thermosetting reaction media and the thermoset appears to create a desirable synergistic cohesive and adhesive effect to the reaction product. Whether or not the proper viscoelastomeric thermoset polymerizate structure and a sufficient amount of plasticizer have been achieved may be ascertained by determining whether or not an adhesion release strength of more than 300 grams-force per square centimeter (g_{f} /cm²) has been achieved. Excessive plasticizer concentration exceeding the thermoset viscoelastomeric loading capacity will typically lead to unbounded plasticizer visible upon the viscoelastomeric reaction product surface. Conversely, an inappropriate diol to triol reaction media ratio will fail to provide the necessary polymerizate structure needed to achieve an adhesion release strength of more than 300 g_{f} /cm². Typically a highly functional adhesive thermoset viscoelastomeric insert **30** will characteristically possess the uniquely distinctive adhesive and cohesive attributes as mentioned herein. By providing the appropriate cross-linkage sites to polyether straight chain linkage with an appropriate plasticizer loading, adhesion release strengths or separation values of more than 400 g_{f}/cm² and typically greater than about 500 g_{f} /cm² may be effectively achieved.

In essence the diols and triols useful in preparing the thermoset reaction products of a desired polymerizate structure are derived from oxygen containing hydrocarbyl diols and triols of a molecular weight of at least 1,000 and include repetitive oxygen containing functional groups such as provided by polyester and polyether groupings. Due to the oxygen electron scavaging effect to satisfy its **2s** orbital needs, it appears that these repetitive internal oxygen containing groupings sandwiched between cross-linkages provide countless polarized enclaves for uniformly hosting corresponding massive polarized plasticizer concentrations within the polymeric structure to collectively create a synergistic cohesive and adhesive effect thereupon. Although the aforementioned viscoelastomeric reaction media primarily centers about viscoelastomers prepared from a thermosetting reaction media incorporating a balanced cross-linkage triol and diol ratio with the cyano reactant in the presence of a sufficient amount and type of plasticizer to provide the desired adhesive and cohesive insert, it is contemplated that this technology may also apply to other thermoset viscoelastomeric producing reaction products having a properly balanced cross-linked molecular structure separated by a proper amount of straight chain polyoxy linkages and polarity in the presence of a sufficient adhesive contributing plasticizer to yield a thermoset viscoelastomeric reaction product insert **30** of a comparable stable cohesiveness, adhesiveness and releasability. In general, the thermosetting viscoelastomeric reaction media is accordingly formulated with the appropriate level of cross-linkage and straight chain reactants containing a sufficient amount of a plasticizer to create the unique highly cohesive and adhesive viscoelastomeric insert **30.** A viscoelastomeric reaction media favoring a substantially lesser cross-linked viscoelastomer loaded with a lesser amount of epoxidized vegetable oil plasticizer had been found to unexpectedly dramatically increase the adhesiveness and cohesiveness of the reaction product. It has also been discovered that other commonly used plasticizing agents for plastics which are unreactive with the viscoelastomeric reaction media reactants may also be effectively used to impart a high degree of adhesiveness and cohesiveness to the reaction product. Such plasticizing agents in conjunction with the epoxidized vegetable oil plasticizer (especially ESO) may be effectively used to impart enhanced tensile strength and softness while also providing a thermosetting reaction media of an exceptional viscosity and workability in the manufacture of the inventive inserts **30.**

The most appropriate adhesive strength for any application will depend to a certain degree upon the particular item **40** to be stowed. The cohesiveness and adhesiveness of the thermoset viscoelastomer and insert **30** may be adjusted by plasticizer type and its loading to fit the item **40** to be stowed. Typically the adhesion release strength may be selectively predetermined prior to the thermosetting reaction product preparation so as to match its intended or desired end use. The adhesion release strength may be regulated by the amount and type of plasticizer used and the polymeric configuration of the thermoset viscoelastomer achieved by the curing of the appropriate reaction media polymerizates. The molecular polymeric structure of the thermoset viscoelastomer may be modified by the cross-linkage density and straight chain linkage present in the thermoset reaction product so as to enable the polymeric structure to house an effective cohesive and adhesive quantum of plasticizer.

The desired adhesion release strength for any inventive insert **30** will depend largely upon the type of container combination **10** and item **40** to be stowed therein. The size, delicacy, configuration and weight of the stowed item **40** will generally establish which releasable adhesive strength value is best suited for any particular end use or stowed item **40.** Fragile items **40,** such as Christmas tree decorations, glass, medication, and the like, will normally require a lesser degree of adhesiveness (*e.g*., 300-400 g_{f} /cm²) which will allow for a less forceful release of the fragile item **40** without damage. Similarly, a heavy hammer **40** may not be adhesively matched by an excessively adhesive insert **30** if the force required to separate the hammer **40** from the insert **30** would make it manually very difficult to separate the hammer **40** from the container **20** or the insert **30.** This excessive adhesiveness may cause the container **20** as well as the adhesively stowed hammer **40** to be lifted together without any hammer **40** separation from the insert **30.** However for certain applications, an insert **30** having a high degree of adhesive strength may be particularly desirable. For example, certain toss games or other grounded devices necessitating a firm grounding of the gaming device would allow for the use of an extremely high adhesive release strength (*e.g.,* 1,200-1,600 g_{f}/cm²). Since the thermoset viscoelastomeric reaction product characteristically possesses an extremely high internal cohesiveness which prevents its internal separation, the thermoset viscoelastomers are uniquely useful for diverse needs requiring a releasable, high tensile strength adhesive which fully retains its structural integrity upon release.

As evident from the aforementioned, the most suitable adhesive strength for the inserts **30** and the thermoset viscoelastomeric reaction product ultimately depends upon its intended end usage. The adhesive strength, cohesiveness, the thermoset reaction media attributes, the compositional uniformity, releasability, antimicrobial characteristics, and a host of other factors, are attributes which uniquely distinguish the inventive inserts 30 and the cured thermoset viscoelastomeric reaction product from all other adhesives. For most applications, an insert **30** and the cured thermoset viscoelastomeric reaction product will normally have an adhesive release strength ranging from more than 300 g_{f}/cm² to about 1,200 g_{f}/cm² and most typically within the range of about 500 g_{f}/cm² to about 1,000 g_{f}/cm². As previously mentioned, substantially higher adhesive strengths may be achieved but may have a more limited utility.

By providing the proper thermosetting polymerizable precursors in the appropriate amounts, the basic thermoset viscoelastomeric chain provides a basic molecular structure especially adaptable for *in situ* loading with plasticizer which, depending upon type, can be used to beneficially alter the adhesive and cohesive characteristics of the cured viscoelastomer. An effective amount of long chain polyether polymeric linkages coupled with the appropriate isocyanate and triol cross-linkages provide a polymeric chain of a desired polarity having an unexpectedly high affinity for a loading of polar plasticizing components within the thermoset viscoelastomeric reaction product infrastructure. The selection of the particular plasticizing agent and its concentration can have a pronounced effect upon the adhesiveness and cohesiveness of the thermoset viscoelastomeric reaction product. This may be exemplified by the loading of the viscoelastomeric reaction product with ester plasticizing agents which are typically made by reacting an alcohol with a fatty acid. The ester plasticizers include the ester condensation product of an alcohol (C₁-C₁₈) with a polycarboxilic acid(s) which esters can be used to an advantage in preparing a thermosetting reaction media and thermoset viscoelastomeric reaction product having an adhesiveness tailor-made for a particular end use. For example, certain applications will require a tenacious adhesiveness whereas other applications are best matched with milder releasable adhesive characteristics, such as for fragile items **40.** Similarly the type of plasticizer (e.g., dibutyl sebecate) may be used to advantage in preparing an uncured reaction media having an exceptional low initial viscosity rendering the uncured reaction media especially suitable for reaction product prefabrication. The polar strength (often referenced as "dipole moment") of these plasticizing esters depends to a certain degree upon the alcohol condensation reactant chain length which also has an effect upon the adhesive characteristic of the thermoset viscoelastomeric reaction product.

Although the thermoset viscoelastomeric structure is especially adapted to loading with a host of plasticizers, plasticizers having a polar attraction to the viscoelastomeric polymeric structure are particularly applicable. This molecular electronic attraction apparently contributes to the unique adhesive and cohesive properties within the reaction product. Since the plasticizers are not effectively loadable into a cured thermoset viscoelastomer, the plasticizing reagents are necessarily uniformly incorporated into the thermosetting viscoelastomeric reaction media to achieve the desired uniform and homogeneous distribution thereof throughout its entire uncured composition. The thermosetting conditions apparently uniformly align the polymerizate reactants with an effective synergistic polar positioning and alignment of the polar attracting molecular chain sections of the thermoset reactants with the plasticizer to provide for a highly effective polarized plasticizer loading and alignment therewithin. This permits a tenacious and cohesive loading of plasticizer without any evidence of plasticizer seepage or separation from its hosting thermoset viscoelastomeric polymeric structure.

In general plasticizers which are suitable as plasticizing agents for polyvinyl chlorides are generally applicable for use as reaction media plasticizers herein. Plasticizers of a higher dipole moment (e.g., dibutyl sebacatehaving a dipole moment of 2.48 debyes (D)) will tend to impart certain desired properties to the polymerizate. Illustrative of plasticizing agents which may be combined with the thermosetting reaction media include the ester plasticizers such as sebacates, adipates, terephthalates, dibenzoates, glutarates, phthalates, azelates, etc. of the C₁-C₁₈ chain ester type. However, different blends of the ester plasticizers may be coblended into the thermosetting reaction media to create or modify the effective working viscosity as in the curing or cured form. For unexpectedly superior adhesiveness, cohesiveness and workability plasticizers of a dipole moment of more than 1.5 D and typically more than 2.0 D may be utilized for this purpose. Epoxidized triglycerides such as the epoxidized animal and vegetable oils are especially effective as a plasticizer component in the thermosetting viscoelastomeric reaction media and especially at levels typically less than 40% by weight of the total reaction media weight with significantly over-all improvements being generally accomplished when the epoxidized triglyceride content is less than 35% by weight and more appropriately less than 30% by weight of the total thermosetting reaction media weight. The incorporation of a lower molecular weight ester plasticizer (e.g., less than 400 MW) (such as an polyalkylene ester plasticizer) in combination with epoxidized triglyceride plasticizer can be utilized to provide an easier fabricating form of the reaction media without adversely affecting its desirable thermoset properties.

Illustrative ester plasticizing agents of a high dipole moment include the dibutyl, dimethyl, and diethyl esters of sebacates, the adipates, the isophthalates, the phathalates, the maleates, the azelates, the glutarates, etc. The total plasticizer concentration will most suitably range from about 20% to about 45% by weight and most typically range from about 25% to about 40% by weight with the weight ratio of epoxidized triglyceride plasticizer to non-epoxidized plasticizer typically ranging from about 1:0 to about 1:3 and most typically from about 1:1 to about 3:1.

The term "cohesive" and "adhesive" herein refer to their common technological meaning. As used herein, the terms "cohesive" and "cohesiveness" refer to the thermoset viscoelastomer cohesive ability to retain its structural integrity when subjected to separating forces. The reaction product and insert cohesive attributes are further reflected by its tensile strength, adhesive separation without leaving any viscoelastomer residue and its elasticity. In contrast, as used herein, the terms "adhesive" and "adhesiveness" refer to the bonding strength of the thermoset viscoelastomer to items **40** adhering to its adhesive surface. The inventive thermoset viscoelastomeric reaction product and the inventive inserts **30** possess an unexpectedly superior stabilization and a powerful adhesiveness which is releasable by a counteracting force overcoming its adhesiveness.

As typical with most thermosetting resins, it cannot be precisely ascertained what phenomena actually occurs when an uncured viscoelastomeric reaction media is cured and rigidly bound to a compatible substrate. Whether the thermosetting creates a chemical bonding or an extremely strong molecular or mechanical bonding is unclear. Nonetheless, it has been discovered that an increase in the diol to triol weight ratio coupled with a reduced plasticizer content will result in a highly tenacious cured bonding of the viscoelastomeric reaction product to a compatible substrate as well as those substrates recognized as being resistant to bonding. In certain applications, a peelable or more readily releasable cured coating would be desired whereas in others a more tenacious bonding is better suited to its end use. Although the curing of the reaction media with the reduced cross-linking triol and plasticizer content affects its supportive base **20A** bonding characteristics, the opposing cured surface area (*i.e.,* unbounded surface) still retains all of its desired cohesive and adhesive characteristics for insert **30** usage. A reduced triol and plasticizer content also increases tensile strength, while providing a more permanent bonding to the supportive structure upon which it was cured.

### EXAMPLES

### Example 1

A thermoset viscoelastomeric reaction product adapted to provide a thermoset viscoelastomeric insert **30** having exceptional adhesive, cohesive and releasability efficacy was prepared by uniformly admixing together a two-part thermosetting reaction media mix comprised of the constituents as shown in **Table 1** below:

**Table 1**

| **A- Mix: Ingredients** | **Percent by Weight:** |
|---|---|
| • Methylene diphenyl diisocyanate based glycol prepolymer (ELASTOCAST TQZP23 available from BASF Corporation) | 6.42% |
| • Epoxidized soybean oil | 26.9% |
| • Dibutyl sebecate | 8.97% |
| | |

| **B- Mix: Ingredients** | **Percent by Weight** |
|---|---|
| • Polyurethane precursor mix formulated polyether triol (ELASTOCAST C-4057 avilable from BASF Corporation) | 28.53% |
| • Polyether diol (ELASTOCAST C-4018 available from BASF Corporation) | 27.72% |
| • Epoxidized soybean oil | 0.00% |
| • Catalyst (COSCAT 83 available from Vertellus Specialties) | 0.16% |
| • UV inhibitor (Tinuvin B75) | 1.30% |
| **Total** | 100% |

### Iteration 1

The uncured admixture was then tested using the Time Sensitivity Test procedure (described above). More particularly, the uncured admixture was then spread evenly at a uniform 4 mm thickness upon a polyvinylchloride (PVC) apron and allowed to cure to the desired thermoset viscoelastomeric reaction product. It was observed that the resultant cured reaction product film was removable from the PVC. The reaction product film was then cut into test panel patch sizes for testing using the testing apparatus depicted in Fig. 5 with the testing procedure being conducted in accordance with the Adhesive and Cohesive Test procedure (set forth above), and additionally performing the optional *time dependent properties* steps set forth therein.

Test samples **130** prepared from the thermoset viscoelastomeric reaction product formulation of this Example 1 consistently provided stabilized test sample results requiring a separation force (*i.e.,* adhesion release strength) of more than 300 g_{f} /cm² to separate from the tested samples **130** from the contact surface **145** of the testing cylinder **140** using the Adhesive and Cohesive Test procedure. Upon performing the optional *time dependent properties* steps, the results of the effect of time after a test sample's initial adhesion to the contact surface **145** of the testing cylinder **140** at the designated time intervals is set forth in **Table 2** below:

**Table 2**

| **Time Sensitive Control** | |
|---|---|
| **Contact Time (sec)** | **Removal Force (g_{f})** |
| 5 | 557 g_{f} /cm² |
| 10 | 545 g_{f} /cm² |
| 15 | 534 g_{f} /cm² |
| 30 | 523 g_{f} /cm² |
| 60 | 591 g_{f} /cm² |
| 300 | 545 g_{f} /cm² |
| 600 | 557 g_{f} /cm² |
| 900 | 580 g_{f} /cm² |

It can be seen that the test sample of this Example 1 reached more than 90% of its maximum adhesive release strength within one (1) minute after its initial contact with the contact surface **145** of the testing cylinder **140.** It has been consistently observed that other test samples of a 300 g_{f} /cm² plus adhesion release strength will typically also reach more than 90% of their maximum adhesive release strength within one (1) minute after each sample's initial contact with the contact surface **145** of the testing cylinder **140.**

Unlike many adhesives which undesirably undergo substantial increases in adhesiveness over prolonged stowing intervals, rendering them generally unfit for use as a releasable adhesive, the reaction product of this Example 1 exhibited excellent stability over extended time periods. Indeed, even after a 6 week test period, the Example 1 reaction product exhibited less than a 10% change in adhesion release strength relative to the adhesion release strength tests taken 60 seconds after initial contact with the contact surface **145** of the testing cylinder **140** of the apparatus **100** illustrated in Fig. 5. As evident from the aforementioned, the tested reaction product (as well as in the other reaction products disclosed herein) characteristically exhibited excellent adhesion and cohesion stability rendering it well suited for use as an adhesive insert **30** for the inventive container combinations **10** herein. Comparatively, it was observed that the dibutyl sebacate plasticizer containing reaction media of this Example 1 substantially reduced the initial uncured viscosity, whereas the 45.84-percent by weight epoxidized soybean oil formulation of Example 2 (discussed below) was more viscous. The more fluid thermosetting reaction media rendered the dibutyl sebacate containing reaction media better suited for many fabrication techniques commonly used to convert the reaction media and the resultant reaction product into a fabricated commercial product. Uniform dispersal of the plasticizer within the unique thermoset polymerizate structure places constraints upon the use of diluting solvent based systems while the application of higher temperatures prematurely hasten curing. Similarly, the polyether and polyester diols and triols of an excessive molecular weight are typically a solid or are waxy, which limits their effective prefabrication usage.

### Iteration 2 - Effect of Pressure

In order to test the effect of an initial application of pressure and essentially a nominal pressure application by the contact surface **145** of the testing cylinder **140** of the apparatus **100** illustrated in Fig. 5 upon test samples **130,** the optional *pressure dependent properties* of the Adhesive and Cohesive Test were performed. More particularly, an applied force of 110 grams of weight and essentially a nominal application force of comparative tests were conducted in order to ascertain the effect pressure had upon adhesion. The test samples **130** were secured to the movable test platform **160** after which a uniform pressure of 110 g_{f} was applied to the testing cylinder **140** to test the pressurized test samples **130.** Similar comparative tests were conducted using the same test apparatus **100** and procedure, except only a nominal pressure (relying solely upon the free hanging cylinder **140** weight of 20 grams) was applied to the test samples **130.** The testing apparatus **100** with the test samples **130** secured to the test platform **160** were then subjected to a constant speed (2.7 cm/sec) withdrawing force using the testing apparatus **100** shown in Fig. 5. The counteracting force needed to break or separate the adhesive bond (*i.e.,* adhesion release strength) between each of the test samples **130** and the cylinder **140** were then determined.

It was observed that the application of the 110 grams of pressure by the testing cylinder **140** upon the tested samples **130** initially resulted in a quicker and higher adhesion release strength (*e.g*., within 15 seconds) than the adhesive release strength of the nominal applied pressure tests which took a substantially longer period of time to obtain their maximum adhesive release strength. However, the nominal applied pressure to cylinder **140** tests ultimately achieved an adhesion release strength comparable to the 110 grams applied pressure sample tests. Obviously the adhesive forces in the nominal pressure applied test caused the viscoelastomeric reaction product test sample **130** to internally undergo further adhesive attractive forces without any other extrinsic factor being attributable to its increase in adhesiveness. This subsequent increase in adhesiveness for the nominal pressurized test application tends to indicate that an internal polarity change within the tested reaction product after initial contact with the contact surface **145** of the testing cylinder **140** may have occurred, which phenomenon may be due to electron migration within the thermoset viscoelastomeric mass after the initial contact of the cylinder **140** to the sample **130.** This delayed adhesive attraction may also possibly arise by reason that the sample **130** draws the cylinder **140** deeper into the sample **130** creating a greater adhesiveness, or some other unknown phenomenon may exist.

### Iteration 3 - Thermosetting Bonding Characteristics

An uncured admixture of this example was then spread evenly at a uniform 3 mm thickness upon a polyvinylchloride film and allowed to cure to the desired thermoset viscoelastomeric reaction product which after separation from the PVC film was then cut into insert test panel patch sizes for testing using the testing apparatus **100** depicted in Fig. 5. The resultant cured reaction product coating exhibited an increased bonding strength between the cured reaction product and the PVC film. The PVC film could nonetheless be manually separated from the reaction product film. By peeling the cured reaction product from the PVC film, the entire PVC film could accordingly be removed from the thermoset viscoelastomeric reaction product film. By covering both surfaces of the thermoset reaction product with protective plastic films (*e.g.,* PVC films) or other plastics to sandwich the reaction product therebetween, a ready-to-use and protected insertable insert **30,130** was formed. Thus, an insert 30,**130** requiring only a stripping of the overlaying protective films (useful from formation of the reaction product insert **30,130** to its ultimate insertion into a container **20)** may be effectively provided for consumer use. The reduced plasticizer level of less than 40% by weight of the reaction media weight tends to enhance the bonding attributes of the cured reaction product coating. The outwardly and unbound surface of the reaction product test sample however possessed excellent stowing attributes. This example illustrates the versatility in preparing reaction products and inserts **30** which permanently bond to a diverse range of base supports **20A** including those which do not normally bond to urethane thermosets.

### Iteration 4 - Permanent Coating Bonding

A freshly uncured reaction media of this Example was used to coat a high density polypropylene supportive base **20A** (*see e.g.,* Figs. 1-3). Three (3) mm of coating was applied to a preflamed bed **22** of a polypropylene fishing tackle box **20.** The reaction media was allowed to cure and bond to the bed **22.** The resulting cured reaction product insert **30** had virtually all of the characteristics of a chemically or permanently bonded coating which could not be effectively removed from the polypropylene container bed **22.** However, the opposite unbound reaction product surface retained its excellent stabilized adhesion separation strength rendering it particularly useful as a permanent insert **30** for the stowable container combination **10.** This reaction media is especially useful to permanently bond an insert **30** to a host of common thermoplastics.

### Iteration 5 - Plasticizer Variations

Using the basic formulation of this Example 1, the weight amount of the plasticizer was held constant but the weight ratio of epoxidized soybean oil (ESO) plasticizer to dibutyl sebecate (DBS) plasticizer was changed to 1:0 (*i.e.,* all ESO), 1:1 (*i.e.,* 50% ESO and 50% DBS) and 1:3 (*i.e.,* 25% ESO and 75% DBS). The 1-ESO to 0-DBS, the 1-ESO to 1-DBS and the 1-ESO to 1-DBS weight ratios. Upon adhesive release strength testing, it was observed that the samples provided values of 568 g_{f} /cm² , 420 g_{f} /cm² and 341 g_{f} /cm², respectively. It may accordingly be observed that by altering the ratios and type of plasticizer, the adhesive character, the adhesive use and the fabricating properties may be preselected to fit any desired particular end use and purpose.

### Example 2

A viscoelastomeric reaction product containing 45.84-percent by weight epoxidized soybean oil plasticizer was prepared by uniformly admixing together a two part thermosetting reaction media mix comprised of the constituents as shown in **Table 3** below:

**Table 3**

| **A- Mix: Ingredients** | **Percent by Weight:** |
|---|---|
| • Methylene diphenyl diisocyanate based glycol prepolymer (ELASTOCAST TQZP23 by BASF Corporation) | 5.56% |
| • Epoxidized soybean oil | 44.44% |
| | |

| **B- Mix: Ingredients** | **Percent by Weight:** |
|---|---|
| • Polyurethane precursor mix formulated polyether triol (ELASTOCAST C-4057 by BASF Corporation) | 28.0% |
| • Polyether diol (ELASTOCAST C-4018 by BASF Corporation) | 17.52% |
| • Epoxidized soybean oil | 1.40% |
| • Catalyst (COSCAT 83) | 0.52% |
| • UV inhibitor (Tinuvin B75) | 1.12% |
| • Dye | 1.40% |
| **Total** | 100% |

### Iteration 1 - Thermosetting Bonding Characteristics

The reaction product was prepared by applying a 3 mm thick coating of the reaction media admixture upon a 0.3 mm PVC film and a 0.3 mm high density polypropylene film and was then cured. It was readily apparent that the cured reaction product coatings were more loosely bound to the PVC film since the film was more easily peelable from the reaction product coating than was the coating of Example 1. The adhesive release strength, when cured *in situ* with the films, was somewhat greater than the maximum adhesive release strength of 490 g_{f}/cm² recorded by the test samples of the uncured reaction product.

The reduced plasticizer content, along with a balanced diol to triol ratio, appears to significantly contribute to the bonding strength of the cured reaction media to a host of supportive structures **20A** when cured *in situ.* For certain applications a bonded but readily peelable *in situ* cured reaction media coating and protective film covering both surfaces of the cured reaction product insert **30** may be commercially desirable. This can provide an insert **30** which may be transported in the channels of commerce and installed in the stowing container combination by the ultimate consumer.

*In situ* cured inserts **30** which are protected on both surfaces by the protective film may be most suitably formulated with about 40-percent by weight to less than 50-percent by weight plasticizer. At plasticizer levels below the 40-percent level the peelablility factor becomes more difficult.

While the foregoing description, figures and examples are directed toward non-limiting exemplary embodiments in accordance with the present invention, it should be appreciated that numerous modifications can be made to each of the individual steps of the methods and the components of the embodiments. Indeed, such modifications are encouraged to be made in the steps, materials, structures and arrangements of the disclosed steps and embodiments of the present invention as defined by the claims.

Thus, the foregoing description of the non-limiting exemplary preferred steps and embodiments should be taken by way of illustration rather than by way of limitation with respect to the present invention as defined by the claims set forth hereinbelow.

## Claims

1. A stable, adhesive and cohesive thermoset viscoelastomeric reaction product prepared by thermosetting a reaction media comprising a substantially uniform admixture of:
A. an isocyanate prepolymer,
B. 35-percent to 55-percent by weight polyols consisting essentially of straight chain linking diols and cross-linking triols; and
C. an effective amount of plasticizer which is less than 50-percent by weight epoxidized triglyceride plasticizer;
wherein:
(i) the diols and triols each comprise repetitive oxygen containing functional groups;
(ii) the diols and triols are present in a weight ratio of 7:13 to 13:7: and
(iii) the effective amount of plasticizer within the reaction media being sufficient to provide an adhesion release strength of more than 300 grams of force per square centimeter, as measured according to the "Adhesive and Cohesive Test" disclosed herein.

2. The reaction product of claim 1, comprising:
A. 3-percent to 10-percent by weight of an isocyanate prepolymer:
B. 10-percent to 40-percent by weight of a polyether triol having molecular weight of at least 1000;
C. 10-percent to 35-percent by weight of a polyether diol having a molecular weight of at least 1000 with the weight ratio of polyether diol to polyether triol ranging from 7:13 to 13:7; and
D. 20-percent to less than 50-percent by weight of a plasticizer

3. The reaction product of claim 1 or claim 2, wherein the isocyanate comprises diisocyanate.

4. The reaction product of any one of claims 1 to 3, wherein the diols comprise polyether diol and the triols comprise polyether triol.

5. The reaction product of claim 4, wherein:
(i) the polyether diol and the polyether triol comprise a polyoxyalkylene grouping selected from the group consisting of polyoxyethylene and polyoxypropylene; and
(ii) the polyether diol and the polyether triol each have a molecular weight of 1,000 to 20,000.

6. The reaction product of claim 2, wherein the reaction media further comprises an epoxidized triglyceride plasticizer to ester plasticizer weight ratio from about 1:3 to about 3:1.

7. The reaction product of any one of claims 1 to 6, wherein:
(i) the epoxidized triglyceride plasticizer comprises epoxidized vegetable oil selected from the group consisting of: soybean oil, castor oil, corn oil, cotton seed oil, perilla oil, safflower oil, linseed oil and tall oil; and
(ii) the ester plasticizer is selected from the group consisting of C₁-C₁₈ chain esters of: sebacate, dibutyl sebacate, adipate, glutarate, dibenzoate, phthalate, terephthalate and azelates.

8. The reaction product of claim 2, wherein the ester plasticizer comprises a molecular weight of less than 500 and a dipole moment of at least 1.5 D.

9. The reaction product of any one of claims 1 to 8, wherein the reaction product is in the form of an insert having a thickness of less than 4 mm.

10. The reaction product of any one of claims 1 to 9, wherein the reaction product further comprises aseptic properties.

11. A method for preparing a stable, adhesive and cohesive thermoset viscoelastomeric reaction product, the method comprising:
A. providing a substantially uniform uncured thermosetting reaction media admixture comprising:
a) an isocyanate prepolymer;
b) 35-percent to 55-percent by weight polyols consisting essentially of straight chain linking diols and cross-linking triols, each having repetitive oxygen-containing functional groups;
c) an effective amount of plasticizer which is less than 50-percent by weight epoxidized triglyceride plasticizer; and
d) a lower molecular weight ester plasticizer of less than 400 MW ;
B. forming the thermosetting reaction media to a desired prefabricated article form; and
C. curing the reaction media to provide thermoset viscoelastomeric reaction product of the desired prefabricated article form,
wherein the reaction media further comprises a diol to triol weight ratio of 7:13 to 13:7, and wherein the effective amount of plasticizer within the reaction media is sufficient to provide an adhesion release strength of more than 300 grams of force per square centimeter, as measured according to the "Adhesive and Cohesive Test" disclosed herein.

12. The method of claim 11, wherein:
A. the isocyanate prepolymer is present in an amount of 3-percent to 10-percent by weight;
B. the triols comprise polyether triol which is present in an amount of 10-percent to 40-percent by weight and the polyether triol has a molecular weight of at least 1000;
C. the diols comprise polyether diol which is present in an amount of 10-percent to 35-percent by weight and the polyether diol has a molecular weight of at least 1000 with the weight ratio of polyether diol to polyether triol ranging from 7:13 to 13:7; and
D. the plasticizer is present in an amount of 20-percent to less than 50-percent by weight.

13. The method of claim 11 or claim 12, wherein:
(i) the isocyanate prepolymer comprises diisocyanate;
(ii) the diols comprise polyether diol;
(iii) the triols comprise polyether triol;
(iv) the epoxidized triglyceride plasticizer comprises an epoxidized vegetable oil plasticizer; and
(v) the ester plasticizer comprises a C₁-C₁₈ chain ester of sebacate.

14. The method of any one of claims 11 to 13, wherein the reaction product is an insert.

15. The method of claim 14, wherein the polyether diol and the polyether triol constitute a polyoxyalkylene grouping selected from the group consisting of polyoxyethylene and polyoxypropylene of a molecular weight ranging from 2000 to 10,000; and wherein the reaction product comprises an insert having a thickness of less than 4 mm.

16. A storage container combination possessing cohesive and adhesive properties to adhesively engage a stowable item at a set position and to release the stowable item from the set position, comprising:
A. a supportive base having a sufficient supportive structure to support a stowable item placed thereupon, and
an insert consisting essentially of a stable, cohesive and adhesive thermoset viscoelastomeric reaction product according to any one of claims 1 to 15.

17. The storage container combination of claim 16, wherein the insert has been disposed onto the supportive base, and wherein the insert comprises sufficient adhesiveness and cohesiveness to retain the stowable item at a stabilized position and to release the stowable item upon application of a force sufficient to overcome an adhesive attraction of the stowable item to the insert.

18. The storage container combination of claim 16, wherein the insert is adhesively affixed to the container or is chemically bonded by curing the reaction media *in situ* to the container.

19. The storage container combination of any one of claims 16 to 18, wherein the container further comprises multiple sidewalls dividing the container bed into multiple compartmentalized sections.

20. The storage container combination of any one of claims 16 to 19, wherein the container is flexible.

21. The storage container combination of any one of claims 16 to 20, wherein the supportive base comprises a substantially flat supportive base without any additional substantial confining structure.

22. The storage container combination of any one of claims 16 to 21, wherein the container combination comprises a fishing tackle box.

23. The storage container combination of any one of claims 16 to 22, wherein the insert imparts antimicrobial properties to the storage container combination.

24. The storage container combination of any one of claims 16 to 23, wherein the isocyanate comprises diisocyanate, wherein the epoxidized triglyceride plasticizer comprises epoxidized soybean oil, and wherein the ester plasticizer comprises a C₁-C₁₈ chain ester of sebacate.

25. A method for preparing a container combination having an aseptic surface and a sufficient stable adhesiveness and cohesiveness to retain and release a stowed item placed thereupon, the method comprising:
A. providing a container comprising a supportive base having a sufficient size and structural support to support a stowed item, and
B. placing a cohesive and adhesive thermoset viscoelastomeric insert in an interfacing relationship to the surface of the supportive base with the insert compositionally comprising a thermoset viscoelastomeric reaction product , wherein the reaction product is formed from a reaction media comprising:
a) 3-percent to 10-percent by weight of an isocyanate prepolymer:
b) 10-percent to 40-percent by weight of a polyether triol having molecular weight of at least 1000;
c) 10-percent to 35-percent by weight of a polyether diol having a molecular weight of at least 1000 with the weight ratio of polyether diol to polyether triol ranging from 7:13 to 13:7;
d) 20-percent to less than 50-percent by weight of an epoxidized triglyceride plasticizer, with said polyether diol providing sufficient straight chain linkage to permit a cohesive loading of an effective amount of plasticizer within the reaction product to provide an adhesion release strength of more than 300 g_{f}/cm^{2;} and
e) an effective amount of a catalytic thermosetting catalyst for curing the thermosetting reaction media.

26. The method of claim 25, wherein the method includes initially coating a surface of the supportive base with the reaction media and thereafter allowing the reaction media to cure *in situ* to form the reaction product.

27. The method of claim 25, wherein the method includes forming the reaction product into a prefabricated insert prior to disposing the reaction product onto the supportive base.

28. The method of any one of claims 25 to 27, wherein:
(i) the isocyanate prepolymer comprises diisocyanate;
(ii) the diol comprises polyether diol;
(iii) the triol comprises polyether triol;
(iv) the epoxidized triglyceride plasticizer comprises epoxidized vegetable oil; and
(v) the ester plasticizer comprises a C₁-C₁₈ chain ester of sebacate.

## Patentansprüche

1. Stabiles, adhäsives und kohäsives wärmehärtendes viskoelastomeres Reaktionsprodukt, das durch Wärmehärten eines Reaktionsmediums hergestellt wird, das eine im Wesentlichen gleichmäßige Beimischung umfasst aus:
A. einem Isocyanatpräpolymer,
B. zu 35 Gewichtsprozent bis 55 Gewichtsprozent Polyolen, die im Wesentlichen aus geradkettigen Verknüpfungsdiolen und Vernetzungstriolen bestehen; und
C. einer wirksamen Menge an Weichmacher, die zu weniger als 50 Gewichtsprozent epoxidierter Triglyceridweichmacher ist;
wobei:
(i) die Diole und Triole jeweils sich wiederholende sauerstoffhaltige funktionelle Gruppen umfassen;
(ii) die Diole und Triole in einem Gewichtsverhältnis von 7:13 bis 13:7 vorhanden sind; und
(iii) wobei die wirksame Menge an Weichmacher innerhalb des Reaktionsmediums ausreichend ist, um eine Adhäsionsablösekraft von mehr als 300 Gramm-Kraft pro Quadratzentimeter bereitzustellen, wie gemäß in der hierin offenbarten "Adhäsions- und Kohäsionsprüfung" gemessen.

2. Reaktionsprodukt nach Anspruch 1, umfassend:
A. zu 3 Gewichtsprozent bis 10 Gewichtsprozent ein Isocyanatpräpolymer;
B. zu 10 Gewichtsprozent bis 40 Gewichtsprozent ein Polyethertriol mit einem Molekulargewicht von mindestens 1000;
C. zu 10 Gewichtsprozent bis 35 Gewichtsprozent ein Polyetherdiol mit einem Molekulargewicht von mindestens 1000, wobei das Gewichtsverhältnis von Polyetherdiol zu Polyethertriol im Bereich von 7:13 bis 13:7 liegt; und
D. zu 20 Gewichtsprozent bis weniger als 50 Gewichtsprozent einen Weichmacher.

3. Reaktionsprodukt nach Anspruch 1 oder Anspruch 2, wobei das Isocyanat Diisocyanat umfasst.

4. Reaktionsprodukt nach einem der Ansprüche 1 bis 3, wobei die Diole Polyetherdiol umfassen und die Triole Polyethertriol umfassen.

5. Reaktionsprodukt nach Anspruch 4, wobei:
(i) das Polyetherdiol und das Polyethertriol eine Polyoxyalkylengruppierung umfassen, die aus der Gruppe ausgewählt ist, die aus Polyoxyethylen und Polyoxypropylen besteht; und
(ii) das Polyetherdiol und das Polyethertriol jeweils ein Molekulargewicht von 1.000 bis 20.000 aufweisen.

6. Reaktionsprodukt nach Anspruch 2, wobei das Reaktionsmedium ferner ein Gewichtsverhältnis von epoxidiertem Triglyceridweichmacher zu Esterweichmacher von etwa 1:3 bis etwa 3:1 umfasst.

7. Reaktionsprodukt nach einem der Ansprüche 1 bis 6, wobei:
(i) der epoxidierte Triglyceridweichmacher epoxidiertes Pflanzenöl umfasst, das aus der Gruppe ausgewählt ist, die besteht aus: Sojabohnenöl, Rizinusöl, Maisöl, Baumwollsaatöl, Perillaöl, Distelöl, Leinöl und Tallöl; und
(ii) der Esterweichmacher aus der Gruppe ausgewählt ist, die aus C₁-C₁₈-kettigen Estern besteht von: Sebacat, Dibutylsebacat, Adipat, Glutarat, Dibenzoat, Phthalat, Terephthalat und Azelaten.

8. Reaktionsprodukt nach Anspruch 2, wobei der Esterweichmacher ein Molekulargewicht von weniger als 500 und ein Dipolmoment von mindestens 1,5 D umfasst.

9. Reaktionsprodukt nach einem der Ansprüche 1 bis 8, wobei das Reaktionsprodukt in Form eines Einsatzes vorliegt, der eine Dicke von weniger als 4 mm aufweist.

10. Reaktionsprodukt nach einem der Ansprüche 1 bis 9, wobei das Reaktionsprodukt ferner aseptische Eigenschaften umfasst.

11. Verfahren zum Herstellen eines stabilen, adhäsiven und kohäsiven wärmehärtenden viskoelastomeren Reaktionsprodukts, wobei das Verfahren umfasst:
A. Bereitstellen einer im Wesentlichen gleichmäßigen ungehärteten wärmehärtenden Reaktionsmedienbeimischung, umfassend:
a) ein Isocyanatpräpolymer,
b) zu 35 Gewichtsprozent bis 55 Gewichtsprozent Polyole, die im Wesentlichen aus geradkettigen Verknüpfungsdiolen und Vernetzungstriolen bestehen, die jeweils sich wiederholende sauerstoffhaltige funktionelle Gruppen aufweisen;
c) eine wirksame Menge an Weichmacher, die zu weniger als 50 Gewichtsprozent epoxidierter Triglyceridweichmacher ist; und
d) einen niedermolekularen Esterweichmacher von weniger als 400 MW;
B. Bilden des wärmehärtenden Reaktionsmediums zu einer gewünschten vorgefertigten Erzeugnisform; und
C. Härten des Reaktionsmediums, um ein wärmegehärtetes viskoelastomeres Reaktionsprodukt der gewünschten vorgefertigten Erzeugnisform bereitzustellen, wobei das Reaktionsmedium ferner ein Diol-zu-Triol-Gewichtsverhältnis von 7:13 bis 13:7 umfasst, und wobei die wirksame Menge an Weichmacher innerhalb des Reaktionsmediums ausreichend ist, um eine Adhäsionsablösekraft von mehr als 300 Gramm-Kraft pro Quadratzentimeter bereitzustellen, wie gemäß in der hierin offenbarten "Adhäsions- und Kohäsionsprüfung" gemessen.

12. Verfahren nach Anspruch 11, wobei:
A. das Isocyanatpräpolymer in einer Menge von 3 Gewichtsprozent bis 10 Gewichtsprozent vorhanden ist;
B. die Triole Polyethertriol umfassen, das in einer Menge von 10 Gewichtsprozent bis 40 Gewichtsprozent vorhanden ist, und das Polyethertriol ein Molekulargewicht von mindestens 1000 aufweist;
C. die Diole Polyetherdiol umfassen, das in einer Menge von 10 Gewichtsprozent bis 35 Gewichtsprozent vorhanden ist, und das Polyetherdiol ein Molekulargewicht von mindestens 1000 aufweist, wobei das Gewichtsverhältnis von Polyetherdiol zu Polyethertriol im Bereich von 7:13 bis 13:7 liegt; und
D. der Weichmacher in einer Menge von 20 Gewichtsprozent bis weniger als 50 Gewichtsprozent vorhanden ist.

13. Verfahren nach Anspruch 11 oder Anspruch 12, wobei:
(i) das Isocyanatpräpolymer Diisocyanat umfasst;
(ii) die Diole Polyetherdiol umfassen;
(iii) die Triole Polyethertriol umfassen;
(iv) der epoxidierte Triglyceridweichmacher einen epoxidierten Pflanzenölweichmacher umfasst; und
(v) der Esterweichmacher einen C₁-C₁₈-kettigen Ester von Sebacat umfasst.

14. Verfahren nach einem der Ansprüche 11 bis 13, wobei das Reaktionsprodukt ein Einsatz ist.

15. Verfahren nach Anspruch 14, wobei das Polyetherdiol und das Polyethertriol eine Polyoxyalkylengruppierung darstellen, die aus der Gruppe ausgewählt ist, die aus Polyoxyethylen und Polyoxypropylen eines Molekulargewichts im Bereich von 2000 bis 10.000 besteht; und
wobei das Reaktionsprodukt einen Einsatz umfasst, der eine Dicke von weniger als 4 mm aufweist.

16. Aufbewahrungsbehälterkombination, die kohäsive und adhäsive Eigenschaften aufweist, um ein verstaubares Element an einer festgelegten Position adhäsiv in Eingriff zu nehmen und das verstaubare Element aus der festgelegten Position zu lösen, umfassend:
A. eine Stützbasis mit einer ausreichenden Stützstruktur, um ein darauf platziertes verstaubares Element zu stützen, und
einen Einsatz, der im Wesentlichen aus einem stabilen, kohäsiven und adhäsiven wärmehärtenden viskoelastomeren Reaktionsprodukt nach einem der Ansprüche 1 bis 15 besteht.

17. Aufbewahrungsbehälterkombination nach Anspruch 16, wobei der Einsatz auf der Stützbasis angeordnet wurde und wobei der Einsatz ein ausreichendes Adhäsionsvermögen und Kohäsionsvermögen umfasst, um das verstaubare Element in einer stabilisierten Position zu halten und das verstaubare Element bei Ausübung einer Kraft zu lösen, die ausreichend ist, um eine Adhäsionsanziehung des verstaubaren Elements zu dem Einsatz zu überwinden.

18. Aufbewahrungsbehälterkombination nach Anspruch 16, wobei der Einsatz adhäsiv an dem Behälter befestigt ist oder durch Härten des Reaktionsmediums in situ chemisch mit dem Behälter verbunden ist.

19. Aufbewahrungsbehälterkombination nach einem der Ansprüche 16 bis 18, wobei der Behälter ferner mehrere Seitenwände umfasst, die das Behälterbett in mehrere unterteilte Abschnitte unterteilen.

20. Aufbewahrungsbehälterkombination nach einem der Ansprüche 16 bis 19, wobei der Behälter flexibel ist.

21. Aufbewahrungsbehälterkombination nach einem der Ansprüche 16 bis 20, wobei die Stützbasis eine im Wesentlichen flache Stützbasis ohne eine zusätzliche wesentliche Begrenzungsstruktur umfasst.

22. Aufbewahrungsbehälterkombination nach einem der Ansprüche 16 bis 21, wobei die Behälterkombination einen Angelspinnerkasten umfasst.

23. Aufbewahrungsbehälterkombination nach einem der Ansprüche 16 bis 22, wobei der Einsatz der Aufbewahrungsbehälterkombination antimikrobielle Eigenschaften verleiht.

24. Speicherbehälterkombination nach einem der Ansprüche 16 bis 23, wobei das Isocyanat Diisocyanat umfasst, wobei der epoxidierte Triglyceridweichmacher epoxidiertes Sojabohnenöl umfasst und wobei der Esterweichmacher einen C₁-C₁₈-kettigen Ester von Sebacat umfasst.

25. Verfahren zum Herstellen einer Behälterkombination, die eine aseptische Oberfläche und ein ausreichendes stabiles Adhäsionsvermögen und Kohäsionsvermögen aufweist, um einen darauf platzierten verstauten Gegenstand zu halten und zu lösen, wobei das Verfahren umfasst:
A. Bereitstellen eines Behälters, umfassend eine Stützbasis, die eine ausreichende Größe und strukturelle Stütze aufweist, um ein verstautes Element zu stützen, und
B. Platzieren eines kohäsiven und adhäsiven wärmehärtenden viskoelastomeren Einsatzes in einer Grenzflächenbeziehung zu der Oberfläche der Stützbasis, wobei der Einsatz in seiner Zusammensetzung ein wärmehärtendes viskoelastomeres Reaktionsprodukt umfasst, wobei das Reaktionsprodukt aus einem Reaktionsmedium gebildet ist, das umfasst:
a) zu 3 Gewichtsprozent bis 10 Gewichtsprozent ein Isocyanatpräpolymer;
b) zu 10 Gewichtsprozent bis 40 Gewichtsprozent ein Polyethertriol mit einem Molekulargewicht von mindestens 1000;
c) zu 10 Gewichtsprozent bis 35 Gewichtsprozent ein Polyetherdiol mit einem Molekulargewicht von mindestens 1000, wobei das Gewichtsverhältnis von Polyetherdiol zu Polyethertriol im Bereich von 7:13 bis 13:7 liegt;
d) zu 20 Gewichtsprozent bis weniger als 50 Gewichtsprozent einen epoxidierten Triglyceridweichmacher, wobei das Polyetherdiol eine ausreichende geradkettige Verknüpfung bereitstellt, um eine kohäsive Beladung einer wirksamen Menge an Weichmacher innerhalb des Reaktionsprodukts zu ermöglichen, um eine Adhäsionsablösekraft von mehr als 300 gf/cm² bereitzustellen; und
e) eine wirksame Menge eines katalytischen wärmehärtenden Katalysators zum Aushärten des wärmehärtenden Reaktionsmediums.

26. Verfahren nach Anspruch 25, wobei das Verfahren anfänglich das Beschichten einer Oberfläche der Stützbasis mit dem Reaktionsmedium und danach das Ermöglichen, dass das Reaktionsmedium *in situ* unter Bildung des Reaktionsprodukts aushärtet, umfasst.

27. Verfahren nach Anspruch 25, wobei das Verfahren das Bilden des Reaktionsprodukts zu einem vorgefertigten Einsatz vor dem Anordnen des Reaktionsprodukts auf der Stützbasis umfasst.

28. Verfahren nach einem der Ansprüche 25 bis 27, wobei:
(i) das Isocyanatpräpolymer Diisocyanat umfasst;
(ii) das Diol Polyetherdiol umfasst;
(iii) das Triol Polyethertriol umfasst;
(iv) der epoxidierte Triglyceridweichmacher epoxidiertes Pflanzenöl umfasst; und
(v) der Esterweichmacher einen C₁-C₁₈-kettigen Ester von Sebacat umfasst.

## Revendications

1. Produit de réaction viscoélastomère thermodurci, stable, adhésif et cohésif, préparé par thermodurcissement d'un milieu de réaction comprenant un mélange sensiblement uniforme de :
A. un prépolymère d'isocyanate,
B. 35 pour cent à 55 pour cent en poids de polyols constitués essentiellement de diols de liaison à chaîne droite et de triols de réticulation ; et
C. une quantité efficace de plastifiant qui est inférieure à 50 pour cent en poids de plastifiant triglycéride époxydé ;
dans lequel :
(i) les diols et les triols comprennent chacun des groupes fonctionnels contenant de l'oxygène répétitifs ;
(ii) les diols et les triols sont présents dans un rapport pondéral de 7:13 à 13:7 ; et
(iii) la quantité efficace de plastifiant dans le milieu de réaction étant suffisante pour donner une force de libération par adhésion de plus de 300 grammes de force par centimètre carré, telle que mesurée selon l'« Essai d'adhésion et de cohésion » divulgué ici.

2. Produit de réaction selon la revendication 1, comprenant :
A. 3 pour cent à 10 pour cent en poids d'un prépolymère d'isocyanate :
B. 10 pour cent à 40 pour cent en poids d'un polyéther triol ayant une masse moléculaire d'au moins 1000 ;
C. 10 pour cent à 35 pour cent en poids d'un polyéther diol ayant une masse moléculaire d'au moins 1000, le rapport pondéral du polyéther diol au polyéther triol se situant dans la plage de 7:13 à 13:7 ; et
D. 20 pour cent à moins de 50 pour cent en poids d'un plastifiant.

3. Produit de réaction selon la revendication 1 ou la revendication 2, dans lequel l'isocyanate comprend du diisocyanate.

4. Produit de réaction selon l'une quelconque des revendications 1 à 3, dans lequel les diols comprennent du polyéther diol et les triols comprennent du polyéther triol.

5. Produit de réaction selon la revendication 4, dans lequel :
(i) le polyéther diol et le polyéther triol comprennent un groupement polyoxyalkylène choisi dans le groupe constitué par le polyoxyéthylène et le polyoxypropylène ; et
(ii) le polyéther diol et le polyéther triol ont chacun une masse moléculaire de 1 000 à 20 000.

6. Produit de réaction selon la revendication 2, dans lequel le milieu de réaction comprend en outre un rapport pondéral du plastifiant triglycéride époxydé au plastifiant ester d'environ 1:3 à environ 3:1.

7. Produit de réaction selon l'une quelconque des revendications 1 à 6, dans lequel :
(i) le plastifiant triglycéride époxydé comprend une huile végétale époxydée choisie dans le groupe constitué par : l'huile de soja, l'huile de ricin, l'huile de maïs, l'huile de coton, l'huile de pérille, l'huile de carthame, l'huile de lin et le tallöl ; et
(ii) le plastifiant ester est choisi dans le groupe constitué par les esters à chaîne en C₁-C₁₈ de : sébacate, sébacate de dibutyle, adipate, glutarate, dibenzoate, phtalate, téréphtalate et azélates.

8. Produit de réaction selon la revendication 2, dans lequel le plastifiant ester présente une masse moléculaire inférieure à 500 et un moment dipolaire d'au moins 1,5 D.

9. Produit de réaction selon l'une quelconque des revendications 1 à 8, dans lequel le produit de réaction se présente sous la forme d'un insert ayant une épaisseur inférieure à 4 mm.

10. Produit de réaction selon l'une quelconque des revendications 1 à 9, dans lequel le produit de réaction comprend en outre des propriétés aseptiques.

11. Procédé pour préparer un produit de réaction viscoélastomère thermodurci, stable, adhésif et cohésif, le procédé comprenant :
A. la fourniture d'un mélange de milieu de réaction thermodurcissable non durci sensiblement uniforme comprenant :
a) un prépolymère d'isocyanate ;
b) 35 pour cent à 55 pour cent en poids de polyols constitués essentiellement de diols de liaison à chaîne droite et de triols de réticulation, ayant chacun des groupes fonctionnels contenant de l'oxygène répétitifs ;
c) une quantité efficace de plastifiant qui est inférieure à 50 pour cent en poids de plastifiant triglycéride époxydé ; et
d) un plastifiant ester de masse moléculaire inférieure, de moins de 400 ;
B. le façonnage du milieu de réaction thermodurcissable en une forme d'article préfabriqué souhaitée ; et
C. le durcissement du milieu de réaction pour obtenir un produit de réaction viscoélastomère thermodurci de la forme d'article préfabriqué souhaitée, dans lequel le milieu de réaction comprend en outre un rapport pondéral du diol au triol de 7:13 à 13:7, et dans lequel la quantité efficace de plastifiant dans le milieu de réaction est suffisante pour donner une force de libération par adhésion de plus de 300 grammes de force par centimètre carré, telle que mesurée selon l'« Essai d'adhésion et de cohésion » divulgué ici.

12. Procédé selon la revendication 11, dans lequel :
A. le prépolymère d'isocyanate est présent en une quantité de 3 pour cent à 10 pour cent en poids ;
B. les triols comprennent du polyéther triol qui est présent en une quantité de 10 pour cent à 40 pour cent en poids et le polyéther triol a une masse moléculaire d'au moins 1000 ;
C. les diols comprennent du polyéther diol qui est présent en une quantité de 10 pour cent à 35 pour cent en poids et le polyéther diol a une masse moléculaire d'au moins 1000, le rapport pondéral du polyéther diol au polyéther triol se situant dans la plage de 7:13 à 13:7 ; et
D. le plastifiant est présent en une quantité de 20 pour cent à moins de 50 pour cent en poids.

13. Procédé selon la revendication 11 ou la revendication 12, dans lequel :
(i) le prépolymère d'isocyanate comprend du diisocyanate ;
(ii) les diols comprennent du polyéther diol ;
(iii) les triols comprennent du polyéther triol ;
(iv) le plastifiant triglycéride époxydé comprend un plastifiant d'huile végétale époxydée ; et
(v) le plastifiant ester comprend un ester à chaîne en C₁-C₁₈ de sébacate.

14. Procédé selon l'une quelconque des revendications 11 à 13, dans lequel le produit de réaction est un insert.

15. Procédé selon la revendication 14, dans lequel le polyéther diol et le polyéther triol constituent un groupement polyoxyalkylène choisi dans le groupe constitué par le polyoxyéthylène et le polyoxypropylène d'une masse moléculaire se situant dans la plage de 2000 à 10 000 ; et dans lequel le produit de réaction comprend un insert ayant une épaisseur inférieure à 4 mm.

16. Combinaison de récipient de stockage possédant des propriétés cohésives et adhésives pour s'engager de manière adhésive avec un article pouvant être rangé au niveau d'une position définie et pour libérer l'article pouvant être rangé de la position définie, comprenant :
A. une base de support ayant une structure de support suffisante pour supporter un article pouvant être rangé placé sur celle-ci, et
un insert constitué essentiellement d'un produit de réaction viscoélastomère thermodurci stable, cohésif et adhésif selon l'une quelconque des revendications 1 à 15.

17. Combinaison de récipient de stockage selon la revendication 16, dans laquelle l'insert a été disposé sur la base de support, et dans laquelle l'insert comprend une adhésivité et une cohésivité suffisantes pour retenir l'article pouvant être rangé au niveau d'une position stabilisée et pour libérer l'article pouvant être rangé lors de l'application d'une force suffisante pour surmonter une attraction adhésive de l'article pouvant être rangé sur l'insert.

18. Combinaison de récipient de stockage selon la revendication 16, dans laquelle l'insert est fixé de manière adhésive au récipient ou est lié chimiquement par durcissement du milieu de réaction in situ sur le récipient.

19. Combinaison de récipient de stockage selon l'une quelconque des revendications 16 à 18, dans laquelle le récipient comprend en outre de multiples parois latérales divisant le lit de récipient en de multiples sections compartimentées.

20. Combinaison de récipient de stockage selon l'une quelconque des revendications 16 à 19, dans laquelle le récipient est flexible.

21. Combinaison de récipient de stockage selon l'une quelconque des revendications 16 à 20, dans laquelle la base de support comprend une base de support sensiblement plane sans aucune structure de confinement substantielle supplémentaire.

22. Combinaison de récipient de stockage selon l'une quelconque des revendications 16 à 21, dans laquelle la combinaison de récipient comprend une boîte à articles de pêche.

23. Combinaison de récipient de stockage selon l'une quelconque des revendications 16 à 22, dans laquelle l'insert confère des propriétés antimicrobiennes à la combinaison de récipient de stockage.

24. Combinaison de récipient de stockage selon l'une quelconque des revendications 16 à 23, dans laquelle l'isocyanate comprend du diisocyanate, dans laquelle le plastifiant triglycéride époxydé comprend de l'huile de soja époxydée, et dans laquelle le plastifiant ester comprend un ester à chaîne en C₁-C₁₈ de sébacate.

25. Procédé pour préparer une combinaison de récipient ayant une surface aseptique et une adhésivité et une cohésivité stables et suffisantes pour retenir et libérer un article rangé placé sur celle-ci, le procédé comprenant :
A. la fourniture d'un récipient comprenant une base de support ayant une taille et un support structurel suffisants pour supporter un article rangé, et
B. le placement d'un insert viscoélastomère thermodurci cohésif et adhésif dans une relation d'interfaçage avec la surface de la base de support, l'insert comprenant, du point de vue de sa composition, un produit de réaction viscoélastomère thermodurci, dans lequel le produit de réaction est formé à partir d'un milieu de réaction comprenant :
a) 3 pour cent à 10 pour cent en poids d'un prépolymère d'isocyanate ;
b) 10 pour cent à 40 pour cent en poids d'un polyéther triol ayant une masse moléculaire d'au moins 1000 ;
c) 10 pour cent à 35 pour cent en poids d'un polyéther diol ayant une masse moléculaire d'au moins 1000, le rapport pondéral du polyéther diol au polyéther triol se situant dans la plage de 7:13 à 13:7 ;
d) 20 pour cent à moins de 50 pour cent en poids d'un plastifiant triglycéride époxydé, ledit polyéther diol fournissant une liaison à chaîne droite suffisante pour permettre une charge cohésive d'une quantité efficace de plastifiant à l'intérieur du produit de réaction pour donner une force de libération par adhésion de plus de 300 gf/cm² ; et
e) une quantité efficace d'un catalyseur de thermodurcissement pour durcir le milieu de réaction thermodurcissable.

26. Procédé selon la revendication 25, dans lequel le procédé comprend le revêtement initial d'une surface de la base de support avec le milieu de réaction et, par la suite, le fait de laisser le milieu de réaction durcir *in situ* pour former le produit de réaction.

27. Procédé selon la revendication 25, dans lequel le procédé comprend le façonnage du produit de réaction en un insert préfabriqué avant de disposer le produit de réaction sur la base de support.

28. Procédé selon l'une quelconque des revendications 25 à 27, dans lequel :
(i) le prépolymère d'isocyanate comprend du diisocyanate ;
(ii) le diol comprend du polyéther diol ;
(iii) le triol comprend du polyéther triol ;
(iv) le plastifiant triglycéride époxydé comprend de l'huile végétale époxydée ; et
(v) le plastifiant ester comprend un ester à chaîne en C₁-C₁₈ de sébacate.
